# EUROPEAN PATENT SPECIFICATION

(11) **EP 2 097 533 B1**
(45) Date of publication and mention of the grant of the patent: **25.08.2010**
(21) Application number: 07858264.0
(22) Date of filing: 28.11.2007
(51) Int. Cl.: C12N 15/86

(54) **VIRAL VECTOR AND USES THEREOF**
VIRALER VEKTOR UND SEINE VERWENDUNGEN
VECTEUR VIRAL ET SES APPLICATIONS

(30) Priority: 28.11.2006 ES 200603036; 03.04.2007 ES 200700882
(43) Date of publication of application: 09.09.2009
(73) Proprietor: Proyecto de Biomedicina Cima, S.L., 31008 Pamplona - Navarra (ES)
(72) Inventor: SMERDOU PICAZO, Cristian, 31008 Pamplona - Navarra (ES); CASALES ZOCO, Erkuden, 31008 Pamplona - Navarra (ES); RODRÍGUEZ MADOZ, Juan Roberto, 31008 Pamplona - Navarra (ES); RAZQUÍN ERRO, Nerea, 31008 Pamplona - Navarra (ES); CUEVAS LABRADOR, Yolanda, 31008 Pamplona (ES); RUIZ GUILLÉN, Marta, 31008 Pamplona - Navarra (ES); PRIETO VALTUEÑA, Jesús, 31008 Pamplona - Navarra (ES)
(74) Representative: ABG Patentes, S.L.
(86) International application number: PCT/ES2007/000688
(87) International publication number: WO 2008/065225

(56) References cited:
- US-B1- 6 458 560
- TAMM KRISTI ET AL: "Mutations in the nuclear localization signal of nsF2 influencing RNA synthesis, protein expression and cytotoxicity of Semliki Forest virus" JOURNAL OF GENERAL VIROLOGY, vol. 89, no. Part 3, March 2008 (2008-03), pages 676-686, XP002484261 ISSN: 0022-1317
- FAZAKERLEY JOHN K ET AL: "A single amino acid change in the nuclear localization sequence of the nsP2 protein affects the neurovirulence of Semliki Forest virus" JOURNAL OF VIROLOGY, vol. 76, no. 1, January 2002 (2002-01), pages 392-396, XP002484262 ISSN: 0022-538X
- RIKKONEN MARJA: "Functional significance of the nuclear-targeting and NTP-binding motifs of Semliki Forest virus nonstructural protein nsP2" VIROLOGY, vol. 218, no. 2, 1996, pages 352-361, XP002484263 ISSN: 0042-6822
- RIKKONEN M ET AL: "NUCLEAR AND NUCLEOLAR TARGETING SIGNALS OF SEMLIKI FOREST VIRUS NONSTRUCTURAL PROTEIN NSP2" VIROLOGY, vol. 189, no. 2, 1992, pages 462-473, XP002484264 ISSN: 0042-6822

## Description

### FIELD OF THE INVENTION

The present invention is comprised within the genetic engineering field. The invention specifically relates to mutated viral vectors and their applications, particularly in *in vitro* stable cell line generation and protein production in a constitutive manner.

### BACKGROUND OF THE INVENTION

Alphavirus expression vectors have been developed from different viruses, including the Sindbis virus (SIN) (34), the Semliki Forest virus (SFV) (18) and the Venezuelan equine encephalitis virus (VEE) (25). Alphavirus vectors are normally based on RNA replicons in which the structural genes have been substituted with a heterologous gene.

The alphavirus replicon contains an ORF encoding the viral replicase (Rep) at its 5' end, which is translated when the RNA is transfected into eukaryotic cells. The Rep is expressed as a polyprotein which is subsequently processed in four subunits (nsps 1 to 4) (30). The unprocessed Rep can copy the RNA vector in a negative RNA strand, a process which only takes place during the first 3 to 4 hours after transfection or infection. Once processed, the Rep will use the negative strand RNA as a mold for synthesizing more replicon molecules. The processed Rep can also recognize an internal sequence in the negative strand RNA, or subgenomic promoter, from which it will synthesize a subgenomic positive strand RNA corresponding to the 3' end of the replicon. This subgenomic RNA will be translated to produce the heterologous protein in large amounts. The replicon RNA can also be packaged into viral particles by means of the co-transfection of cells with one or more auxiliary RNAs which can provide the viral structural proteins in trans (2, 4, 8, 25, 29) or by using stable packaging cell lines (24).

Alphavirus vector replication is cytopathic in most mammalian cells due to mechanisms which are still not completely understood (3, 9, 13). The cytopathic effect induced by these vectors is mediated by p53-independent apoptosis and normally occurs between 48-72 hours after transfection or infection (14). It is suspected that nsp2, one of the 4 components of the viral replicase, could have an important role in apoptosis induction (11, 12).

Cytopathic alphavirus vectors have been used in a series of applications including *in vitro* protein production and characterization (33), as well as in studies about vaccination and in cancer gene therapy (19, 26). However, an important drawback of such wild type viral vectors lies in the fact that they cannot be used in applications in which a long-lasting transgene expression is desired.

To solve this drawback, several groups have identified a series of mutations in the alphavirus replicase which can convert those cytopathic viral vectors into noncytopathic viral vectors, which allows a more long-lasting expression of the recombinant products expressed by the viral vector. These studies have led to the generation of noncytopathic vectors derived from SIN (7, 22), SFV (20-22) and, more recently, from VEE and EEE (Eastern equine encephalitis virus) (23).

Most of the noncytopathic mutations detected in alphaviruses have been located in Rep subunit nsp2. This protein contains a helicase domain at the amino end and a proteolytic domain at the carboxyl end, the latter being involved in the Rep processing in its four subunits (30). It has been shown that the noncytopathic mutations described for nsp2 affect the proteolytic domain of nsp2 or the positions close to the cleavage sites between nsp1/2 or nsp2/3, thus altering the Rep processing.

A noncytopathic mutant isolated in SIN, which contained a single amino acid change (P for L) in position 726 in nsp2 (P726L SIN vector in nsp2), showed Rep hyperprocessing (7). This mutant was able to effectively establish continuous replication in BHK cells. The introduction of different amino acid changes in the same position of this mutant showed that there is a strong positive correlation between the RNA replication level and the cytopathogenicity of the vector. This noncytopathic SIN vector has widely used *in vitro,* because it can provide a long-lasting transgene expression with good stability levels and expression levels which were approximately 4% of those obtained with the [wild type (wt)] original SIN vector (1). Nevertheless, in spite of the fact that said vector is not cytopathic, it lacks the capacity to generate stable cell lines with high expression levels. Indeed, in cell lines generated with the P726L SIN vector carrying the LacZ gene, the transgene expression was lost in 45% of the transfected cells after 5 passages, and the stability of the expression was only achieved by selecting individual clones (1), which considerably delayed obtaining a high number of cells which can efficiently express the transgene. Even so, these lines maintained low expression levels (4% of the levels obtained the wt SIN vector).

A noncytopathic SFV vector comprising the mutations P718T and R649H (R649H/P718T) in subunit nsp2, expressing the puromycin-resistance pac gene, has furthermore been described although its capacity to generate high-expression stable cell lines has not been described (Smerdou, C. et al. 2004. Seventh International Symposium on Positive Strand RNA Viruses, S. Francisco, USA).

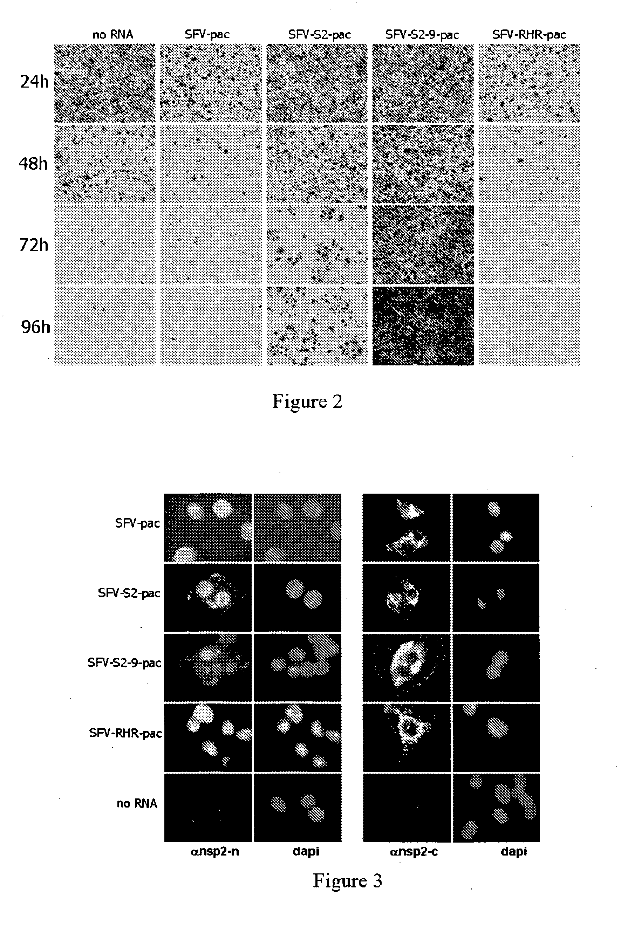

With regard to the noncytopathic SFV mutants described by Perri et al. (22), including mutants SF2A (mutation L10T in nsp2) and SF2C (mutation L713P in nsp2), as well as double mutant PD (S259P and R650D in nsp2) described by Lundström et al. (21), it is true that they can express protein levels that are similar to or even greater than those of the wild type virus (mutant PD), but in all cases these mutants are still cytopathic (see Example 8 of this description) and the generation of stable cell lines expressing heterologous proteins based on said viral vectors has not been described. The data relating to the cytopathic effect of the SFV-PD vector are furthermore backed by results subsequently published by Lundström et al. (20), showing how β-gal or GFP expression in BHK cells infected with an SFV-PD vector carrying LacZ or GFP as a marker gene reaches a maximum after 48 hours to later decrease drastically in the next 3-4 days, which indicates that a cytopathic effect is occurring in the cells (see Figure 2 of the mentioned reference).

Therefore, although point mutants in the gene encoding the nsp2 protein of the alphaviruses SIN and SFV have been described and although these mutants have a decrease in cytopathogenicity, none of said mutants has shown the capacity to generate stable cell lines with high expression levels.

Therefore, there is still a need to develop alternative noncytopathic viral alphavirus vectors, useful for producing stable cell lines which can stably produce the heterologous protein in the presence of selection.

### SUMMARY OF THE INVENTION

Alphavirus vectors can express high recombinant protein levels in different types of cells. However, this expression is transient due to the cytopathic nature of viral replication. To adapt these vectors for long-term studies, noncytopathic mutants of the Sindbis virus (SIN), of the Semliki Forest virus (SFV) and of the Venezuelan equine encephalitis virus (VEE) have been isolated. Most of these mutants contain changes in nsp2, a subunit of the viral replicase.

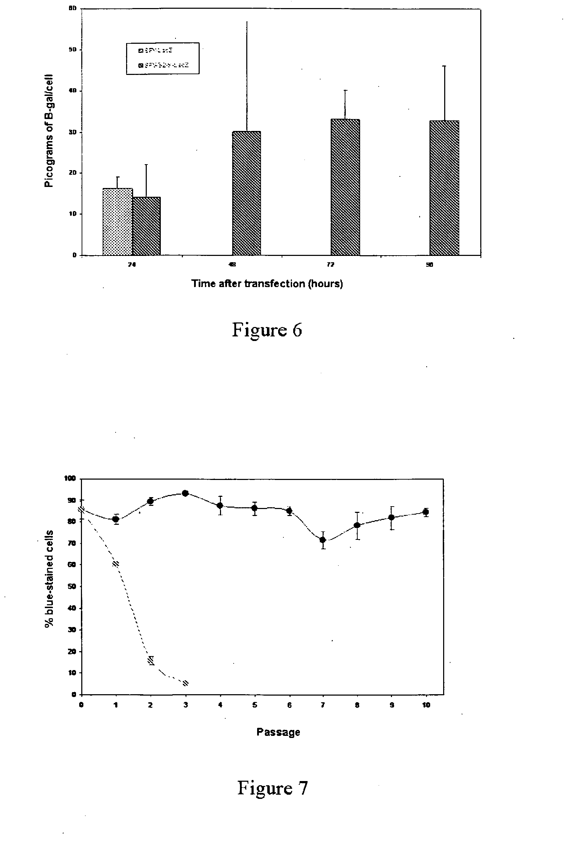

To generate new noncytopathic SFV vectors, the mutations described for creating a noncytopathic SIN vector have been introduced in a conserved position in SFV. Curiously enough, it was found that an SFV replicon containing mutation P718T in nsp2 and carrying LacZ gene as a heterologous gene (SFV-LacZ) gave rise to noncytopathic variants forming colonies expressing β-galactosidase (β-gal) without applying selection to them, despite the fact that it was apparently unable to replicate in most of the cells. The hypothesis was made that that the noncytopathic variants were due to secondary adaptive mutations. To isolate these variants, the puromycin N-acetyltransferase (pac) gene [selection gene] was introduced in mutant SFV-P718T and the BHK cell clones that were puromycin-resistant were selected. A noncytopathic replicon containing a second mutation in the nsp2 nuclear localization signal, specifically mutation R649H was rescued from one of the selected clones. This double mutant, identified as SFV-S2-9 in this description, containing the mutations R649H and P718T in nsp2, replicated at levels that were 60 times less than those of the wild type SFV vector, which fact was correlated to the absence of cytopathogenicity in cells transfected with the SFV vector containing the double mutation R649H/P718T in nsp2. A similar β-gal expression level was observed in cells transfected with SFV-S2-9-LacZ and SFV-LacZ 24 hours after transfection, which was in the range of approximately 15 pg/cell (Figure 6).

Surprisingly enough, noncytopathic viral vectors based on said double SFV mutant (SFV-S2-9) containing the double mutation R649H/P718T in nsp2 were able to generate stable cell lines which can express β-gal for at least 10 passages in culture (the percentage of cells expressing β-gal, determined in each passage by means of the X-gal staining, varied between 70% and 90% according to the passage, but was above 85% in passage 10, which indicated a great stability of the transgene expression in the presence of selection, as can be seen in Figure 7), further being the constitutive expression because it increased over time in cells transfected with SFV-S2-9-LacZ, reaching a stabilization at approximately 30 pg of β-gal/cell 48 hours after transfection. In other words, by means of the transfection with the noncytopathic double mutant SFV vector (R649H/P718T), an expression was quantitatively reached that was two times that achieved 24 hours after transfection when the cells were transfected with the wild type SFV vector.

Said noncytopathic double mutant SFV vector (R649H/P718T) was also able to generate stable cell lines which can constitutively express heterologous proteins of interest, such as rat cardiotrophin-1 (rCT) and the human insulin-like growth factor (IGF-I), at levels similar to those obtained when the wild type SFV vector was used.

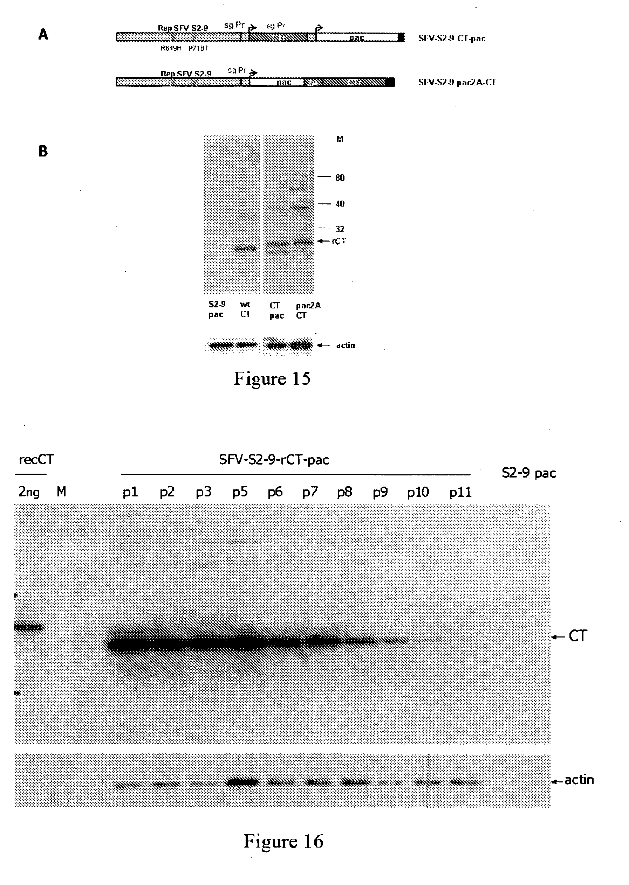

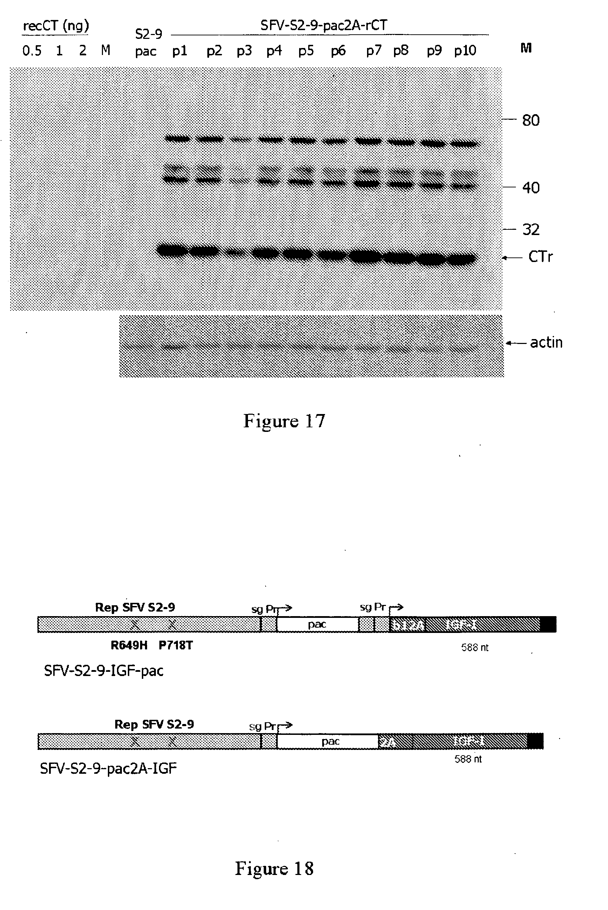

Indeed, said double mutant SFV vector (R649H/P718T) was able to generate stable cell lines which can constitutively express rCT at levels similar to those obtained with the wild type SFV vector, which were approximately 4.3 pg/cell (Example 9). Although a high stability of the vector was observed in the cell lines analyzed, in the lines expressing rCT and pac from 2 independent subgenomic promoters, a lower stability was observed than with the LacZ gene, the rCT expression starting to decrease from passage 6 to virtually disappear at passage 11 (Figure 16). However, the fusion of the rCT and pac genes using the nucleotide sequence encoding foot and mouth disease virus (FMDV) autoprotease 2A as a linker allowed generating stable cell lines in which the stability of the rCT expression remained without variations for at least 10 passages (Figure 17).

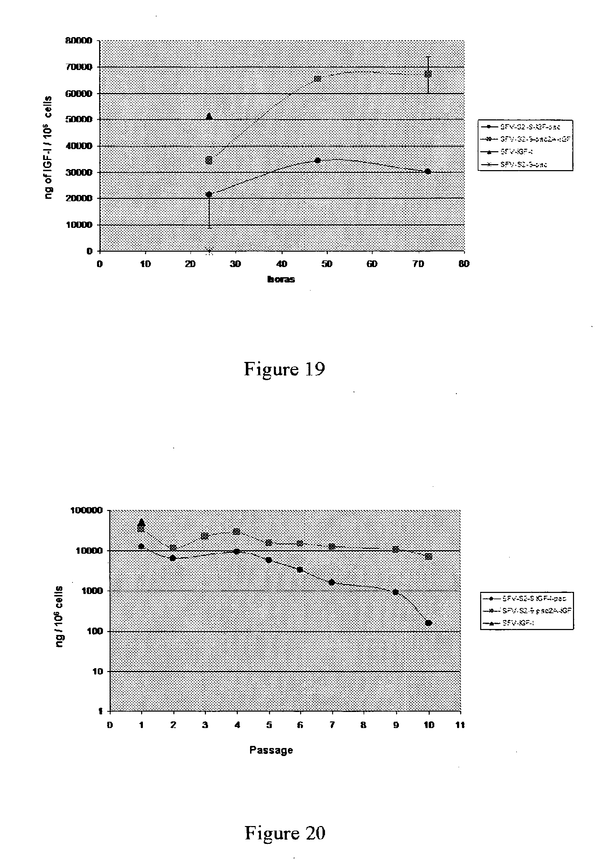

Likewise, said double mutant SFV vector (R649H/P718T) was also able to generate stable cell lines which can express IGF-I at levels that can be compared to those obtained with the wild type SFV vector (Figure 19), which were approximately 50 pg/cell (Example 10). The lines expressing IGF-I and pac from 2 independent subgenomic promoters showed IGF-I expression levels that were somewhat lower than the wild type SFV vector (about 2 times less in passage 1) and further showed a lower stability than with the LacZ gene, the expression starting to decrease from passage 5 and decreasing about 80 times between passages 1 and 10 (Figure 20). The fusion of the pac and IGF-I genes using the nucleotide sequence encoding FMDV autoprotease 2A as a linker allowed generating stable lines in which the IGF-I expression in passage 1 was closer to that of the wild type SFV vector and in which the stability of the IGF-I expression was maintained with small variations until passage 10 (in passage 10 it was only 4 times lower that observed in passage 1) (Figure 20).

In addition, as can be verified in Example 8, the present invention breaks apart a prejudice established in the state of the art as it demonstrates that mutated SFV vectors present in the state of the art and defined as noncytopathic maintain the cytopathogenicity characteristics.

As expressed in the present invention, the term "stable cell line" relates to cell lines in which the percentage of cells expressing a heterologous product (e.g., a peptide or a heterologous protein, etc.) in passage 10 is greater than 85%, said percentage being able to be maintained or exceeded in successive or subsequent passages.

The term "constitutive expression", as used herein, relates to the additional capacity which, surprisingly enough, the aforementioned stable cell lines have to express the heterologous product at a quantitatively high level (greater than 50% with respect to that achieved 24 hours after translation when the cells are translated with the wild type vector).

Likewise, as is known in the state of the art, a "RNA replicon" is a nucleotide sequence replicating unitarily using a complementary RNA as an intermediate which is useful as a mold for producing more molecules of the original RNA. To that end, specific sequences at the RNA ends are generally necessary. The replicon contained in the viral vectors provided by the present invention comprise the 5' end necessary for SFV replication, the sequence encoding the SFV replicase enzyme with mutations P718T and R649H in the nsp2 region, at least one viral SFV subgenomic promoter, a polynucleotide comprising a selection gene, a polynucleotide comprising a nucleotide sequence encoding a heterologous product of interest, and the 3' end necessary for SFV replication containing a terminal sequence of polyadenines (Poly A). In a particular embodiment, said polynucleotide comprising the selection gene, as well as said polynucleotide comprising a nucleotide sequence encoding a heterologous product of interest, are placed downstream of 2 independent subgenomic promoters and operatively linked to said promoters (i.e., one of said polynucleotides is placed downstream of, and operatively linked to a subgenomic promoter and the other polynucleotide is placed downstream of, and operatively linked to the other subgenomic promoter). In another particular embodiment, said polynucleotide comprising the selection gene and said polynucleotide comprising a nucleotide sequence encoding a heterologous product of interest are fused to one another, advantageously through a polynucleotide comprising a nucleotide sequence encoding a post-translational proteolytic cleavage site, advantageously a post-translational autoproteolytic cleavage site, and are placed downstream of and operatively linked to a single subgenomic promoter.

Therefore, in an aspect, the invention relates to a viral vector (viral vector of the invention) comprising a replicon of the Semliki Forest virus (SFV), wherein said replicon comprises (i) the nucleotide sequence encoding the SFV replicase enzyme with mutations P718T and R649H in the nsp2subunit, (ii) a polynucleotide comprising a selection gene, and (iii) a polynucleotide comprising a nucleotide sequence encoding a heterologous product of interest.

In another aspect, the invention relates to the use of a viral vector of the invention for generating *in vitro* stable cell lines which can constitutively express heterologous products of interest.

In another aspect, the invention relates to a stable cell line which can constitutively express heterologous products of interest, characterized in that it is a cell line transfected with a viral vector of the invention.

In another aspect, the invention relates to a method for generating *in vitro* said stable cell line which can constitutively express heterologous products of interest.

In another aspect, the invention relates to the use of said stable cell line for producing *in vitro* heterologous products of interest.

In another aspect, the invention relates to a method for producing *in vitro* a heterologous product of interest which comprises culturing said stable cell line in conditions which allow the expression of the heterologous product of interest contained in the viral vector used to generate said stable cell line.

### BRIEF DESCRIPTION OF THE DRAWINGS

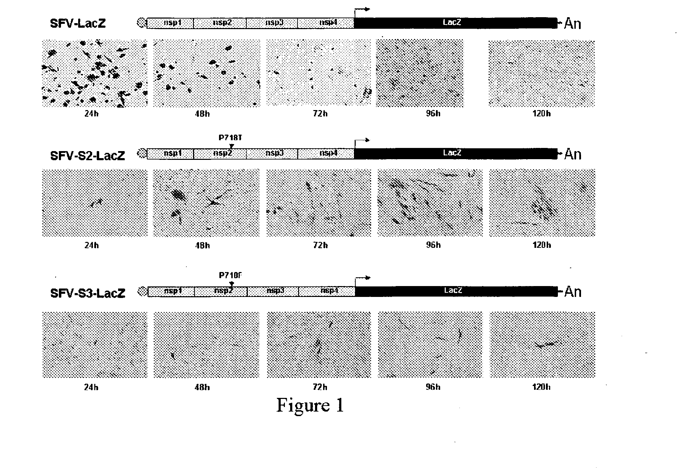

Figure 1. Evaluation of the cytopathogenicity of the SFV vectors carrying SIN derived mutations. Plasmids pSFV-LacZ containing mutations P718T (pSFV-S2-LacZ) and P718F (pSFV-S3-LacZ) in nsp2 were constructed as described in the examples. RNA was transcribed *in vitro* from each of these plasmids as well as from the original pSFV3-LacZ and was electroporated in BHK cells which were seeded in plates with a diameter of 35 mm at low confluence and were stained with X-gal after being cultured for the indicated times. In the case of SFV-S2-LacZ or SFV-S3-LacZ, a 1:5 passage was given to the cells transfected with said vectors after 96 hours in culture. A schematic representation of the RNA of each vector is shown above each set of images. Cap: circle at the left end of the vectors; nsp: nonstructural proteins; horizontal arrow: SFV subgenomic promoter; An: poly A.
Figure 2. Puromycin-resistant BHK cells selected after being electroporated with different RNAs. The BHK cells were electroporated with the indicated SFV RNA vectors or without RNA (no RNA), they were seeded in plates with a diameter of 35 mm and were allowed to recover for 24 hours before added puromycin at 5 µg/ml. The plates were stained with methyl violet in the indicated times after transfection.
Figure 3. Intracellular location of nsp2 in cells transfected with SFV RNA. The BHK cells were electroporated with the SFV RNA vectors or vectors without RNA (no RNA) and were analyzed by means of immunofluorescence 24 hours after transfection. Mouse monoclonal antibodies that were specific for the nuclear (αnsp2-n) or cytoplasmic (αnsp2-c) forms of nsp2 were used as primary antibodies, and a polyclonal rabbit serum specific for FITC-conjugated mouse IgGs was used as a secondary antibody. Dapi-stained nuclei of the same cells were viewed with a UV filter (Dapi).

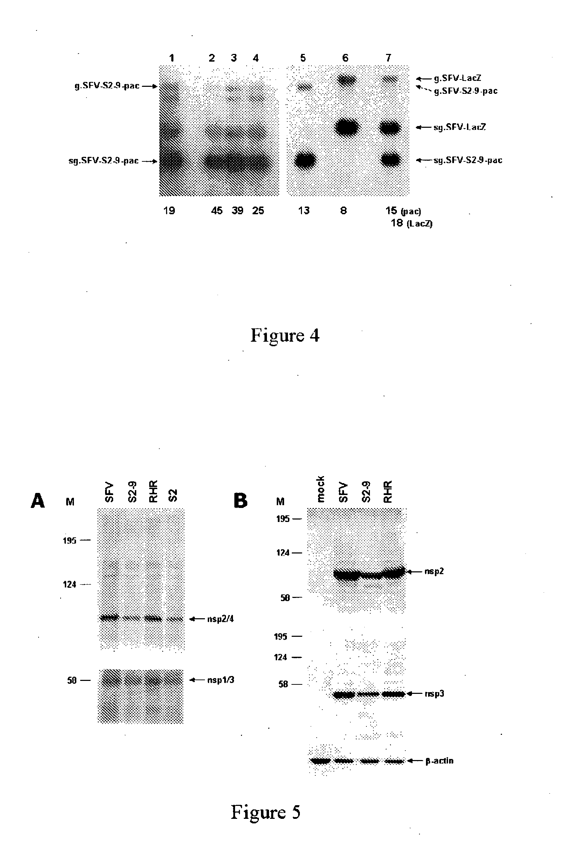

Figure 4. Analysis of the SFV RNA in the transfected BHK cells. The total RNA was extracted from a stable BHK cell line obtained after the electroporation of replicon SFV-S2-9-pac and the selection with puromycin (lane 1) or from BHK cells electroporated with SFV-S2-9-pac (lanes 2-4), SFV-pac (lane 5), SFV-LacZ (lane 6) or coelectroporated with replicons SFV-S2-9-pac and SFV-pac (lane 7) and was analyzed 24 hours (lanes 2, 5 to 7), 48 hours (lane 3) or 72 hours (lane 4) after electroporation. The presence of the genomic (g) and subgenomic (sg) SFV RNAs was analyzed by means of Northern blotting with a ³²P-labelled oligonucleotide specific for the SFV subgenomic promoter. The numbers at the lower part of the gels indicate the subgenomic/genomic (sg/g) RNA ratios in each case. In lane 7, they refer to the sg/g ratio of SFV-S2-9-pac (pac) or of SFV-LacZ (LacZ), respectively. The left part of the gel was exposed for 72 hours whereas the right part was exposed for 1 hour.
Figure 5. *In vitro* and *in vivo* expression of nonstructural proteins. A) The original SFV-pac RNA (wild type) or the SFV-pac RNA containing the indicated mutations were translated in rabbit reticulocyte lysates in the presence of [³⁵S]-methionine and [³⁵S]-cysteine, and were analyzed by means of SDS-PAGE in 8% gels and were later autoradiographed. Two gels containing the same samples were run for different times to obtain a better separation of the bands with a high molecular weight (upper gel, longest migration) and to detect monomers nsp1 and 3 with a smaller molecular weight (lower gel, shortest migration). B) Analysis of the replicase expression in the transfected cells. The lysates of the control BHK cells, of stable BHK cell line obtained after the electroporation with replicon SFV-S2-9-pac and selection with puromycin (S2-9) or of cells electroporated with the original SFV-pac RNA (wild type) or of mutant SFV-RHR-pac (RHR) were analyzed by means of immunoblotting using a polyclonal rabbit antiserum specific against nsp2 (upper gel) or against nsp3 (central gel). The same samples with an antibody specific against β-actin were analyzed as an internal control.
Figure 6. Analysis of the β-gal expression. BHK cells were electroporated with the indicated RNAs and the β-gal expression level per cell was determined at the indicated times after electroporation. The x-axis shows the time after transfection in hours and the γ-axis shows the picograms of β-gal per cell.
Figure 7. Stability of the β-gal expression in the cells transfected with vector SFV-S2-9-LacZ-pac. The BHK cells were electroporated with SFV-S2-9-LacZ-pac RNA and were selected in the presence of puromycin at 5 µg/ml. The selected cells were passaged every 2-3 days with or without puromycin and the percentage of cells expressing β-gal in each passage was determined by means of X-gal staining. The data corresponds to the mean of three different experiments, the standard deviation being shown. The figure shows a typical field of BHK cells containing vector SFV-S2-9-LacZ-pac passaged 10 times in the presence of puromycin and stained with X-gal. The x-axis shows the number of passages and the γ-axis shows the percentage of cells stained blue.

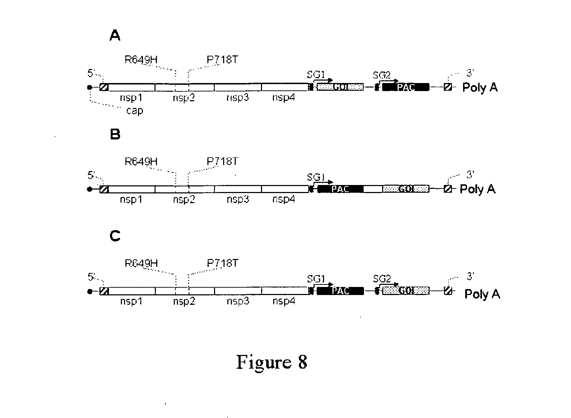

Figure 8. Schematic representation of 2 embodiments, in the form of RNA, of the viral vector for generating stable cell lines and producing stable proteins. The SFV replicon is flanked by the 5' and 3' sequences necessary for SFV replication, in which the 3' sequence incorporates a terminal sequence of polyadenines (Poly A). The replicase with subunits nsp1 to nsp4 and the SFV subgenomic promoter overlapped with nsp4 (SG1) are included inside the replicon. Mutations R649H and P718T typical of the vector replicase are shown on nsp2. The replicon also incorporates a selection gene (PAC) and a gene of interest (GOI) encoding the recombinant protein which is to be constitutively produced. In Examples A and C, the replicon also incorporates a second subgenomic promoter (SG2) such that the PAC and GOI expression is controlled by a different SG promoter, SG1 or SG2 without distinction according to the A or C construct chosen. In Example B, the PAC and GOI expression is controlled by the same SG1 promoter, producing a hybrid protein which is cleaved by proteases in a specific site intercalated between PAC and GOI. cap: cap structure.

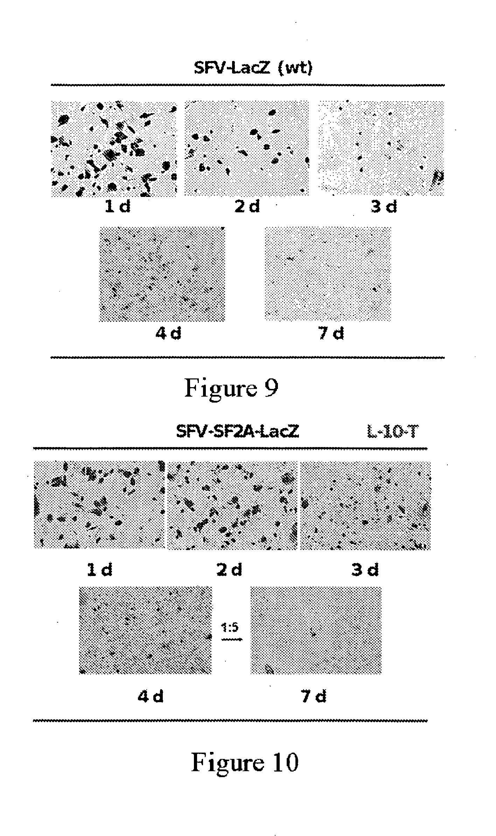

Figure 9. Evaluation of the cytopathic effect of SFV-LacZ. RNA was transcribed *in vitro* from plasmid pSFV-LacZ (wild type vector) and was electroporated in BHK cells. The cells were distributed in 35 mm plates at low confluence and were fixed and stained with X-gal at the indicated times (d, day).
Figure 10. Evaluation of the cytopathic effect of SFV-SF2A-LacZ. RNA was transcribed *in vitro* from plasmid pSFV-SF2A-LacZ and was electroporated in BHK cells. The cells were distributed in 35 mm plates at low confluence and were fixed and stained with X-gal at the indicated times (d, day). The cells were passaged 1:5 four days after transfection.

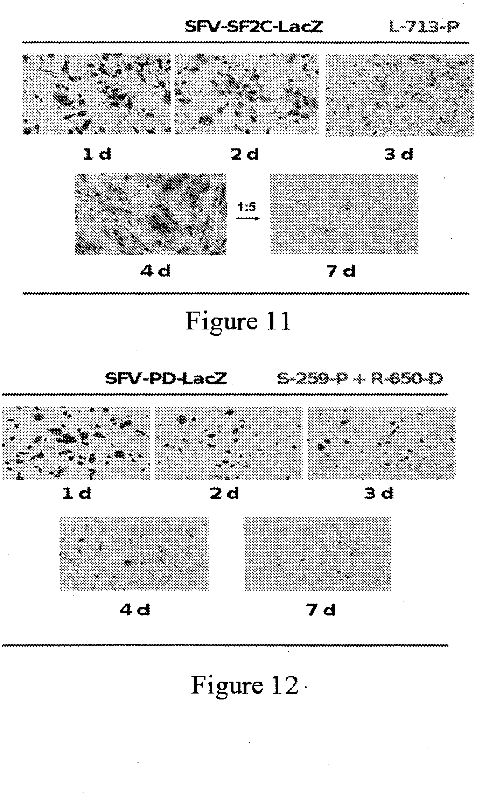

Figure 11. Evaluation of the cytopathic effect of SFV-SF2C-LacZ. RNA was transcribed *in vitro* from plasmid pSFV-SF2C-LacZ and was electroporated in BHK cells. The cells were distributed in 35 mm plates at low confluence and were fixed and stained with X-gal at the indicated times (d, day). The cells were passaged 1:5 four days after transfection.
Figure 12. Evaluation of the cytopathic effect of SFV-PD-LacZ. RNA was transcribed *in vitro* from plasmid pSFV-PD-LacZ and was electroporated in BHK cells. The cells were distributed in 35 mm plates at low confluence and were fixed and stained with X-gal at the indicated times (d, day).

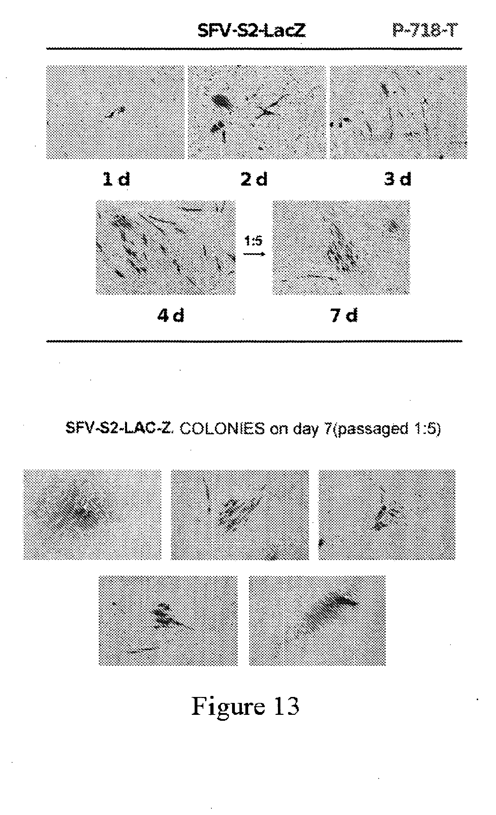

Figure 13. Evaluation of the cytopathic effect of SFV-S2-LacZ. RNA was transcribed *in vitro* from plasmid pSFV-S2-LacZ and was electroporated in BHK cells. The cells were distributed in 35 mm plates at low confluence and were fixed and stained with X-gal at the indicated times (d, day). The cells were passaged 1:5 four days after transfection. This mutant S2 was able to give rise to the formation of colonies of cells expressing β-gal.

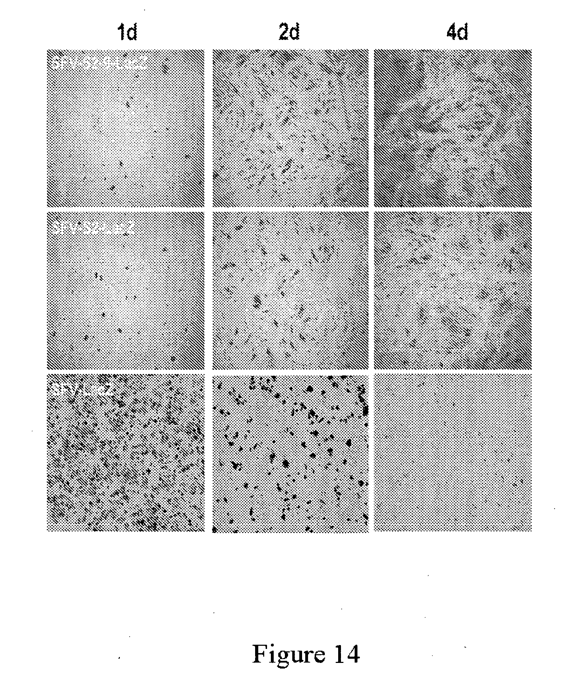

Figure 14. Evaluation of the cytopathic effect of vector SFV-S2-9-LacZ carrying mutations P718T and R649H. RNA was transcribed *in vitro* from plasmid pSFV-S2-9-LacZ, pSFV-S2-LacZ or pSFV-LacZ and was electroporated in BHK cells. The cells were distributed in 35 mm plates at low confluence and were fixed and stained with X-gal at the indicated times (d, day).
Figure 15. Analysis of the rat cardiotrophin-1 (rCT) expression. A) Diagram of the 2 noncytopathic SFV vectors carrying the pac gene and the rCT gene. sg Pr, subgenomic promoter; 2A, nucleotide sequence encoding foot and mouth disease virus (FMDV) autoprotease 2A. B) Analysis of the rCT expression. BHK cells were electroporated with RNA of SFV-S2-9-rCT-pac (rCT-pac), SFV-S2-9-pac2A-rCT (pac-2A-rCT) or SFV-S2-9-pac (S2-9-pac) as a negative control and were selected in the presence of puromycin at 5 µg/ml. The selected cells were lysated and the rCT expression was analyzed by means of immunoblotting using an antibody specific for cardiotrophin (upper gel). As an amount control, the same samples were analyzed with an antibody against actin (lower gel). wtCT, lysate of BHK cells transfected with SFV-rCT obtained 24h after transfection and used as positive control. M, molecular weight markers (kDa).
Figure 16. Analysis of the stability of the rCT expression in BHK cells from vector SFV-S2-9-rCT-pac. The BHK cells were electroporated with SFV-S2-9-rCT-pac RNA and were selected in the presence of puromycin at 5 µg/ml. The selected cells were passaged 1:5 every 2-3 days up to 11 consecutive passages (p1 to p11), the rCT expression in cell lysates being analyzed in each passage by means of immunoblotting with an antibody specific for cardiotrophin (upper gel) and with an antibody specific against actin as a load control (lower gel). Several amounts of purified recombinant rCT (recCT) were used as an amount control. An experiment representative of two conducted experiments that gave a similar expression and stability result is shown. S2-9-pac, negative control of cells electroporated with vector SFV-S2-9-pac. M, molecular weight markers (kDa).
Figure 17. Analysis of the stability of the rCT expression in BHK cells from vector SFV-S2-9-pac2A-rCT. The BHK cells were electroporated with SFV-S2-9-pac2A-rCT RNA and were selected in the presence of puromycin at 5 µg/ml. The selected cells were passaged 1:5 every 2-3 days up to 10 passages (p1 to p10), the rCT expression in cell lysates being analyzed in each passage by means of immunoblotting with an antibody specific for cardiotrophin (upper gel) and with an antibody specific against actin as a load control (lower gel). Several amounts of purified recombinant rCT (recCT) were used as an amount control. An experiment representative of four conducted experiments that gave a similar expression and stability result is shown. S2-9-pac, negative control of cells electroporated with vector-SFV-S2-9-pac. M, molecular weight markers (kDa).
Figure 18. Diagram of the noncytopathic SFV vectors carrying the pac gene and the IGF-I gene. The vector SFV-S2-9-IGF-pac carrying the pac gene and the IGF-I gene under the control of independent viral subgenomic promoters (sg Pr), where the IGF-I gene has been fused with the SFV capsid translation enhancer (b1) using the nucleotide sequence encoding FMDV autoprotease 2A (2A) as a linker. The vector SFV-S2-9-pac2A-IGF carries the pac gene fused with the IGF-I gene using sequence 2A as a linker.
Figure 19. Analysis of the IGF-I expression. BHK cells were electroporated with RNA of SFV-S2-9-IGF-pac, SFV-S2-9-pac2A-IGF or SFV-S2-9-pac (negative control) and were selected in the presence of puromycin at 5 µg/ml. The selected cells were passaged once (passage 1), the supernatant being collected 24, 48 or 72 hours after plating them. The IGF-I expression was analyzed in each of the supernatants by means of an ELISA specific for human IGF-I. SFV-IGF-I, supernatant of BHK cells transfected with RNA of the wild type vector SFV-IGF-I obtained 24h after transfection and used as a positive control.
Figure 20. Analysis of the stability of the IGF-I expression in BHK cells from different vectors. BHK cells were electroporated with RNA of SFV-S2-9-IGF-pac or SFV-S2-9-pac2A-IGF and were selected in the presence of puromycin at 5 µg/ml. The selected cells were passaged every 2-3 days up to 10 consecutive passages, the supernatant being collected in each passage 24 hours after plating them. The IGF-I expression was analyzed in the supernatants of each of the passages by means of an ELISA specific for human IGF-I. SFV-IGF-I, supernatant of BHK cells transfected with RNA of vector SFV-IGF-I obtained 24h after transfection and used as positive control.

### DETAILED DESCRIPTION OF THE INVENTION

### Explanation of the Invention

Alphavirus vectors have several advantages, such as high transgene expression levels, wide tropism and easy handling. The cytopathogenicity caused by these vectors in most vertebrate cells can be advantageous in some applications, such as vaccination and cancer gene therapy, in which the apoptosis of transduced cells can lead to the release of antigens which can be uptaken by immune cells, which would favor an immune response against the expressed recombinant antigen or against tumor antigens, respectively (19). However, the use of these vectors in applications in which a long-lasting transgene expression is needed is hindered by its cytopathic nature.

The possibility of adapting alphavirus vectors for long-term expression has been investigated by different laboratories, which has led to the isolation of noncytopathic variants of SIN, SFV, VEE and EEE (7, 20-23). In most cases, the noncytopathic mutations have been isolated by using vectors carrying a heterologous gene encoding a protein conferring resistance to an antibiotic, such as puromycin or G418. When the cells transfected with this type of vector were incubated in the presence of the antibiotic, only those containing noncytopathic mutant replicons expressing the resistance gene were selected.

Although most of the alphavirus mutants that have been described carry mutations in nsp2, these mutations alter different protein residues in different viruses, in spite of the existence of a high degree of sequence homology between the different alphaviruses. Taking this homology sequence as a basis, the effect of a well characterized SIN mutation, which affected residue 726 of nsp2 in SIN, was investigated in the context of SFV. Mutations P726L and P726T have been described as noncytopathic by Frolov et al. (7) and Perri et al. (22), respectively. In the first study, residue 726 also mutated to all the other possible amino acids, which generated a collection of SIN mutants with different degrees of cytopathogenicity. Frolov et al. were able to establish a correlation between the RNA replication level and cytopathogenicity, concluding that changes in residue 726 of nsp2 which reduced the RNA levels to less than 5% of the levels observed in the wild type vector would produce a noncytopathic phenotype (7).

Two different changes, P726T and P726F, which had been able to reduce the RNA levels in SIN to 5.1% and to 1.6% of the wild levels, respectively, were chosen and the corresponding mutations were introduced in position 718 of SFV nsp2, the mutants identified by the inventors as S2 and S3, respectively, being generated. None of these changes was able to produce a noncytopathic phenotype in SFV by itself. The studies conducted by transfecting BHK cells with those SFV mutants (S2 and S3) in which the LacZ reporter gene had been incorporated demonstrated that only a small percentage of the transfected cells could maintain the vector replication. Mutant S2 (P718T) seemed more stable than S3 (P718F), and gave rise to large colonies of cells expressing β-gal in the absence of selection. It was therefore intuited that these colonies contained replicons with additional adaptive mutations. To select these colonies and identify the possible secondary mutations present in the replicons, the N-acetyltransferase gene (pac) was cloned into SFV mutant S2 and the colonies of the cells cultured in the presence of puromycin were isolated. In one of the clones analyzed, a second mutation was found in position 649 of nsp2 (R649H) which, in combination with mutation P718T, was able to provide a noncytopathic phenotype for the SFV vector based on that double SFV mutant (P718T/R649H). That double SFV mutant (P718T/R649H) was called mutant S2-9 by the inventors and appears indistinctly in this description as mutant SFV-S2-9 or simply S2-9.

Mutation R649H affects the SFV nuclear localization signal ⁶⁴⁸RRR which has been described as being responsible for the partial transport of nsp2 to the cell nucleus (27). The change of Arg for Hys in position 649 constitutes a very conservative change, which could explain the fact that no evident effect is observed on the transport of nsp2 to the nucleus, neither in double mutant S2-9 (P718T/R649H) nor in simple mutant R649H. The latter mutant (R469H) did not show any reduction in the cytopathogenicity when it was compared with the wild type SFV vector, which indicated that both mutations in positions 649 and 718 were necessary to eliminate the cytopathic effect. Fazakerley et al. have described a more drastic change in position 649 (R649D), which led to the attenuation of the neurovirulence of SFV in the context of a complete viral genome (6). In this case, mutation R649D completely interrupted the transport of nsp2 to the nucleus, although it did not suppress the cytopathic effect of the virus on BHK cells. All this data suggested that sequence ⁶⁴⁸RRR of nsp2 could be involved in the cytopathogenicity of the SFV.

Mutant S2-9, containing double mutation P718T/R649H, was able to replicate at levels that were approximately 60 times lower than those observed for the wild type SFV vector, which could explain the absence of cytopathogenicity of said mutant S2-9. The level of subgenomic RNA in mutant S2-9 was only 30 times lower than that of the wild type SFV vector, which indicated that the subgenomic/genomic RNA ratio had increased approximately two-fold in noncytopathic mutant S2-9. This ratio was even greater a short time after transfection, which suggests that the synthesis of genomic RNA in the noncytopathic vector could be delayed in relation to that of subgenomic RNA. Both the decrease of the amount of RNA and the increase of the subgenomic/genomic RNA ratio observed for mutant S2-9 were very similar to those described in the SIN vectors containing noncytopathic mutations in position 726 of nsp2 (7, 22). However, these results were a contradiction to those described by Perri et al. for noncytopathic SFV mutants, in which the amount of mutant RNAs was greater than that of the wild type virus, and in which the subgenomic/genomic RNA ratios also decreased (22), which suggests that different mechanisms could be involved in establishing a long-lasting replication in the SFV mutants isolated by this group. The lower RNA replication level in mutant S2-9 was not due to an effect in cis of the mutations present in the RNA sequence, because a wild type replicase provided in trans by the wild type SFV vector allowed recovering the genomic and subgenomic RNA levels of mutant S2-9 to values close to those of the wild type SFV vector. Several studies conducted with the alphavirus mutants have demonstrated that noncytopathic mutations in nsp2 can alter the proteolytic activity of this protein, which leads either to a replicase hyperprocessing (7), or to a slower processing of this polyprotein (22). In the present invention, clear differences in the replicase processing between mutant S2-9 and the wild type SFV vector were not observed, neither by means of *in vitro* translation, nor by means of immunoblot analysis.

In spite of the low replication levels of mutant S2-9, the amount of β-galactosidase (β-gal) produced by vector SFV-S2-9-LacZ (a viral vector based on SFV mutant S2-9) 24 hours after transfection was very similar to that obtained with a wild type SFV vector expressing the same transgene. The expression level of vector S2-9 increased approximately two-fold at later times after transfection, which is correlated with the increase observed in the RNA levels. The recombinant protein expression level in other described noncytopathic vectors has shown great variability, ranging from only 4% of the wild type expression in the case of the SIN mutant P726L (1) to 1.000% in the case of double mutant SFV-PD developed by Lundström et al (21). However, the noncytopathic nature of this vector was placed in doubt when the same authors showed that β-gal or GFP expression in BHK cells infected with an SFV-PD vector carrying LacZ or GFP as a marker gene reaches a maximum after 48 hours to later decrease drastically in the following 3-4 days (20). In relation to this, it is worth adding that Example 8 of this description breaks apart a prejudice in the state of the art as it demonstrates that the SFV-PD vector developed by Lundström et al (21) is cytopathic. Some additional mutants described by this group containing heat-sensitive mutations were also able to express high recombinant protein levels, but only at the permissive temperature (20, 21). The reason for which mutant S2-9 could express larger amounts of the recombinant protein than the wild type SFV vector having much lower RNA levels could be related to the strong protein synthesis inhibition induced by the latter vector. Two recent publications indicate that eIF2alpha phosphorylation is the mechanism whereby the alphaviruses induce a translation inhibition in the host cell at late times during infection (15, 31). At the same time, these authors demonstrate that the translation enhancer element present at the 5' end of the capsid gene allows an efficient translation of the viral structural polyprotein in the presence of phosphorylated eIF2alpha. It has been demonstrated that when the capsid translation enhancer is fused in phase with the amino end of a recombinant protein in the SFV or SIN vector, it increases the recombinant protein expression level 8-fold (10, 28). This effect was not observed in the context of the viral vector derived from SFV mutant S2-9, probably because translation by eIF2alpha phosphorylation was not being inhibited in the cells transfected with that vector. The absence of protein synthesis inhibition in the cells transfected with the viral vector derived from SFV mutant S2-9 could be responsible for the generation of high expression levels with lower RNA levels than those reached with the wild type SFV vector.

The packaging efficiency of the viral vector derived from SFV mutant S2-9 was approximately 6 logarithmic units lower than that of the wild type SFV vector carrying the same transgene. This could be due to either the low RNA replication levels or to a deficiency in the packaging of the double SFV mutant (S2-9). The packaging experiments in which the RNA of said viral vector derived from the double SFV mutant S2-9 replicated at almost normal levels by means of contransfecting cells with a wild type SFV vector were only able to increase 15-fold the titer of the S2-9 viral particles, which indicated that the low RNA levels were only partially responsible for the low packaging efficiency of this vector. The SFV vector containing mutation R649H only was packaged at levels similar to those of the wild type SFV vector, whereas the SFV vector containing mutation P718T (SFV-S2) only was packaged deficiently. Although the fact that vector SFV-S2 was not able to replicate in most of the transfected cells could be the factor responsible for this low packaging efficiency, everything indicates that this mutation alone or in combination with mutation R649H affects the SFV RNA encapsidation in viral particles. The SFV packaging signals have been mapped in a genome region comprising nucleotides 2767 to 2824 (32), and which does not overlap any of the mutations present in mutant S2-9, located in positions 3643 (R649H) and 3849-51 (P718T). However, it cannot be discarded that these mutations can affect the secondary structure of genomic RNA, altering the accessibility of the packaging signals. More mutagenesis studies are needed to determine the precise function of the sequences in SFV packaging.

It has also been demonstrated that viral vectors derived from mutant S2-9 can be efficiently used to generate stable cell lines of BHK cells constitutively expressing recombinant proteins, without needing to isolate clones. For this purpose, in addition to mutations P718T and R649H, the vector must contain the desired transgene and a selection gene (e.g., a gene encoding a protein conferring resistance to an antibiotic, etc.) useful for selecting cells that have been efficiently transduced. Using LacZ as the reporter transgene and the pac gene as a selection gene, a cell line was generated which showed very high stability, approximately the 85% of the cells expressing β-gal after 10 passages in the culture, and with expression levels which were similar to those of the wild type SFV vector. This contrasts with the observations in a similar cell line generated with a noncytopathic SIN vector carrying mutation P726L in nsp2 and the LacZ gene as a reporter gene and expressing β-gal at levels that are approximately 25 times lower than the wild SIN vector (1). In the latter case, the LacZ expression was lost in 45% of the cells after 5 passages, although a higher stability could be reached with this system if the cell line was derived from a cloned population of cells expressing β-gal.

The viral vector derived from mutant S2-9 was able to generate stable cell lines constitutively expressing rat cardiotrophin-1 (rCT). In this case, the rCT and pac expression from a vector containing 2 subgenomic promoters (SFV-S2-9-rCT-pac) gave rise to the generation of cell lines in which the expression remained stable for the first 5 passages, gradually being lost from passage 5 onwards (Figure 16). However, vector SFV-S2-9-pac2A-rCT in which the pac gene and the gene encoding rCT are fused in phase using the nucleotide sequence encoding FMDV autoprotease 2A as a linker gave rise to the generation of very stable cell line which maintained a high rCT expression similar to that obtained with a wild type SFV vector, for at least 10 passages in culture (Figure 17).

Likewise, the viral vector derived from mutant S2-9 was also able to generate stable cell lines expressing insulin-like growth factor I (IGF-I). In this case, the IGF-I and pac expression from a vector containing 2 subgenomic promoters (SFV-S2-9-IGF-pac) gave rise to the generation of cell lines in which the expression remained stable for the first 4-5 passages, gradually being lost from passage 5 onwards and decreasing 80-fold until reaching passage 10 (Figure 20). However, vector SFV-S2-9-pac2A-IGF in which the pac gene and the gene encoding IGF-I are fused in phase using the nucleotide sequence encoding FMDV autoprotease 2A as a linker gave rise to the generation of cell lines with higher stability which maintained a high IGF-I expression similar to that obtained with a wild type SFV vector, with only an approximate 4-fold decrease in 10 passages in culture (Figure 20). The latter vector (SFV-S2-9-pac2A-IGF) further showed IGF-I expression levels in early passages which were approximately 2 times greater than those observed with vector SFV-S2-9-IGF-pac.

Vector SFV-S2-9-pac can therefore be used to quickly generate stable mammalian cell lines which can produce heterologous products of interest, e.g., recombinant proteins, in large amounts.

### Viral Vector of the Invention

In an aspect, the present invention relates to a viral vector based on the Semliki Forest virus (SFV), hereinafter viral vector of the invention, comprising a replicon of the Semliki Forest virus (SFV), wherein said replicon comprises (i) the nucleotide sequence encoding the SFV replicase enzyme with mutations P718T and R649H in subunit nsp2, (ii) a polynucleotide comprising a selection gene, and (iii) a polynucleotide comprising a nucleotide sequence encoding a heterologous product of interest.

The viral vector of the invention contains an SFV replicon comprising the nucleotide sequence encoding the SFV replicase comprising mutations P718T and R649H in subunit nsp2. Each of these mutations affects a nucleotide triplet, both of them being located in the sequence encoding the subunit or nonstructural protein nsp2 of the viral replicase. Mutation P718T indicates that proline (P), located in position 718 of the amino acid sequence of protein nsp2 encoded by the wild type SFV, is substituted with a threonine (T) in protein nsp2 encoded by the mutated vector (viral vector of the invention). In the same way, mutation R649H indicates that arginine (R) located in position 649 of the amino acid sequence of subunit nsp2 encoded by the wild type SFV, is substituted with a histidine (H) in subunit nsp2 encoded by the mutated vector (viral vector of the invention). Both positions 718 and 649 relate to the position of the substituted amino acid in the already cleaved and processed sequence of subunit nsp2.

In a specific embodiment of the invention, the SFV replicon present in the viral vector of the invention comprises SEQ ID NO: 1 and SEQ ID NO: 2:
- SEQ ID NO: 1 includes the 5' end necessary for SFV replication, the sequence encoding the SFV replicase with mutations P718T and R649H in nsp2, and, overlapping the replicase, a viral SFV subgenomic promoter; and
- SEQ ID NO: 2 includes the 3' end necessary for SFV replication containing a terminal sequence of polyadenines (Poly A).

These two sequences together form the skeleton of the vector and the polynucleotide comprising the selection gene and the polynucleotide comprising the nucleotide sequence encoding a heterologous product of interest are intercalated between them.

The expression of the polynucleotide comprising the selection gene and the expression of the polynucleotide comprising the nucleotide sequence encoding the heterologous product of interest can be controlled by two independent SFV subgenomic promoters or by a single subgenomic promoter controlling the expression of the polynucleotide comprising the selection gene and the expression of the polynucleotide comprising the nucleotide sequence encoding the heterologous product of interest in the form of a fusion protein.

Therefore, in a particular embodiment, the viral vector of the invention comprises:
a) a polynucleotide comprising the nucleotide sequence encoding a heterologous product of interest, the expression of which is controlled by a first SFV subgenomic promoter (SG1); and
b) a polynucleotide comprising a selection gene, the expression of which is controlled by a second SFV subgenomic promoter (SG2).

Said first and second SFV subgenomic promoter can be identical or different. In a specific embodiment, the expression of the selection gene and the expression of the heterologous product of interest are controlled by two identical or different independent SFV subgenomic promoters.

As shown in Figure 8, the polynucleotide comprising the nucleotide sequence encoding a heterologous product of interest and the polynucleotide comprising a selection gene are located inside the SFV replicon, between the subgenomic promoter overlapped in the replicase and the 3' end necessary for SFV replication.

In a particular embodiment, the polynucleotide comprising the nucleotide sequence encoding a heterologous product of interest (GOI, gene of interest) is located downstream of the subgenomic promoter (SG1) overlapped in the replicase and its expression is controlled by said promoter SG1. Another subgenomic promoter SG2 controlling the expression of the polynucleotide comprising the selection gene, which is located downstream of SG2 is then incorporated and its expression is controlled by said promoter SG2 (Figure 8A). As mentioned above, subgenomic promoters SG1 and SG2 can be identical or different.

In another particular embodiment, the viral vector of the invention is also constructed with the same 2 subgenomic promoters(SG1 and SG2), but exchanging the positions of the polynucleotide comprising the nucleotide sequence encoding the heterologous product of interest (GOI) and the polynucleotide comprising the selection gene, such that SG1 controls the expression of the polynucleotide comprising the selection gene and SG2 controls the expression of the polynucleotide comprising the nucleotide sequence encoding the heterologous product of interest (GOI) (Figure 8C). As mentioned above, subgenomic promoters SG1 and SG2 can be identical or different.

In another alternative particular embodiment, the viral vector of the invention comprises, downstream of a subgenomic promoter (e.g., SG1), a construct comprising the polynucleotide comprising the selection gene and the polynucleotide comprising the nucleotide sequence encoding the heterologous product of interest, fused in phase by means of a polynucleotide linker; said linker is advantageously a polynucleotide comprising the nucleotide sequence encoding a post-translational (auto)proteolytic cleavage site.

Virtually any nucleotide sequence encoding a post-translational (auto)proteolytic cleavage site, and consequently the (auto)protease or the amino acid or peptide sequence encoded by said nucleotide sequence, can be used in the context of the present invention.

In a particular embodiment, said linker is a polynucleotide comprising the nucleotide sequence encoding an (auto)protease acting in cis between the proteins resulting from the translation of the selection gene and of the nucleotide sequence encoding the heterologous product of interest; i.e., in a particular embodiment, said linker is a polynucleotide comprising a nucleotide sequence which, when it is translated, provides a cleavage site whereby the expressed fusion protein or polyprotein is post-translationally processed in the proteins forming said fusion protein or polyprotein. Therefore, for the sake of simplicity, the expression "nucleotide sequence encoding a protease" is occasionally used in this description as a synonym of the expression "nucleotide sequence encoding a post-translational (auto)proteolytic cleavage site". By way of a non-limiting illustration, said nucleotide sequence encoding an (auto)protease acting in cis between the proteins resulting from the translation of the selection gene and of the nucleotide sequence encoding the heterologous product of interest, comes from a virus, for example, a picornavirus, an alphavirus, etc. In a particular embodiment, said linker comprises the nucleotide sequence encoding region 2A of the foot and mouth disease virus (FMDV) polyprotein or FMDV autoprotease 2A. In another particular embodiment, said linker comprises the nucleotide sequence encoding the carboxy terminal domain of the SFV capsid with proteolytic activity. A schematic representation of this alternative particular embodiment is shown in Figure 8B, in which it is shown that a subgenomic promoter (e.g., SG1) controls the expression of said construct comprising the polynucleotide comprising the selection gene and the polynucleotide comprising the nucleotide sequence encoding the heterologous product of interest (GOI), fused in phase by means of said linker. The cotranslation (joint translation) of both genes (selection gene and GOI) thus occurs in a single polyprotein which, after the translation, is quickly cleaved by means of rupture in the (auto)proteolytic cleavage site, producing the selection protein and the heterologous product of interest. The use of these post-translational (auto)proteolytic rupture sites has been previously described in European patent application EP 736099 and also by Ryan and Drew (35), particularly the use of the sequence encoding region 2A of the FMDV polyprotein (FMDV autoprotease 2A), the entire content of which is included by reference.

Alternatively, in another particular embodiment, said linker is a polynucleotide comprising the nucleotide sequence encoding a cleavage site for a protease acting in trans; in this case, said protease could be expressed by the cell transfected with the viral vector of the invention, either natively or recombinantly, or alternatively, said protease could be exogenously added to release the heterologous product of interest of the fusion protein comprising the selection protein and the heterologous product of interest. Virtually any nucleotide sequence encoding a cleavage site for a protease acting in trans, and consequently the amino acid sequence encoded by said sequence, can be used in the context of the present invention. By way of a non-limiting illustration, said nucleotide sequence encoding a cleavage site of a protease acting in trans can be a nucleotide sequence encoding an amino acid sequence which can be cleaved by an endopeptidase, etc. By way of a non-limiting illustration, said nucleotide sequence encoding a cleavage site for a protease acting in trans is a nucleotide sequence encoding a cleavage site for a virus protease, a potyvirus for example, such as the Etch Tobacco Virus (ETV), etc. and said protease can be expressed by the cell transfected with the viral vector of the invention (either natively or because it has been suitable transformed), etc.

Alternatively, in another particular embodiment, said linker is a polynucleotide comprising the nucleotide sequence encoding a cleavage site which can be recognized by a chemical reagent, e.g. cyanogen bromide, cleaving in the methionine residues, etc.

In said construct comprising the polynucleotide comprising the selection gene fused in phase to the polynucleotide comprising the nucleotide sequence encoding the heterologous product of interest, the 3' end of the polynucleotide comprising the selection gene can be fused in phase by means of said linker to the 5' end of the polynucleotide comprising the nucleotide sequence encoding the heterologous product of interest (GOI). Alternatively, the 3' end of the polynucleotide comprising the nucleotide sequence encoding the heterologous product of interest (GOI) can be fused in phase by means of said linker to the 5' end of the polynucleotide comprising the selection gene.

When it is expressed by carrier cells, the selection gene present in the viral vector of the invention allows selecting the cells carrying the viral vector of the invention from those cells which have not been transfected or which have lost it. Virtually any selection gene which allows selecting the cells carrying the viral vector of the invention can be used to implement the present invention. By way of a non-limiting illustration, said selection gene can be a gene the expression of which confers resistance to an antibiotic, a gene which allows synthesizing an essential nutrient which is omitted in the culture medium, a gene offering a selective advantage to the transfected cells, etc. In a particular embodiment, said selection gene is a gene the expression of which confers resistance to an antibiotic, a toxic antibiotic for mammalian cells for example, such as the gene conferring hygromycin resistance (hph), the gene conferring neomycin resistance (neoR), the gene encoding the puromycin N-acetyltransferase enzyme (pac), the expression of which confers to the cells carrying the viral vector of the invention resistance to the puromycin antibiotic, etc. The use of the pac gene allows selecting the cells carrying the viral vector of the invention from those which have been transfected or which have lost it, adding puromycin to the medium.

The heterologous product of interest can be virtually any protein or peptide of interest, e.g., a reporter protein or peptide (β-gal, etc.); or a peptide, a protein, or an antibody (or a functional fragment thereof) with therapeutic or diagnostic applications; or any recombinant protein or peptide or interest. As used herein, the term "heterologous" further includes "recombinant", i.e., it does not appear naturally. Illustrative non-limiting examples of heterologous products of interest include peptides and proteins with therapeutic applications, e.g., insulin-like growth factor I (IGF-I), cardiotrophin-1 (CT1), oncostatin M (OSM), interferon alpha (e.g., IFNa5), amphiregulin (AR), glial cell-derived neurotrophic factor (GDNF), endothelial cell protein C/activated protein C receptor (EPCR), antibodies (or functional fragments thereof) of interest or of therapeutic or diagnostic application, etc. Therefore, the polynucleotide comprising the nucleotide sequence encoding the heterologous product of interest present in the viral vector of the invention comprises the sequence encoding the heterologous product of interest.

The "mutated SFV replicase gene - GOI - selection gene" assembly, or, alternatively, the "mutated SFV replicase gene - selection gene - GOI" assembly, flanked by the 5' and 3' sequences necessary for replication, forms an RNA replicon which can be self-amplified inside the cell.

The viral vector of the invention can be used in RNA form or also in DNA form.

When it is used in RNA form, the viral vector of the invention is completed with the addition of a cap structure at its 5' end.

When it is used in DNA form, the complete vector would include a functional promoter in eukaryotes, a cytomegalovirus (CMV) promoter, for example, the SFV replicon sequence in any of the previously defined embodiments, and a transcription termination signal sequence, a signal sequence derived from SV40 for example. The vector is thus transcribed to RNA inside the transfected cells where it will be self-amplified.

Optionally, if desired, the viral vector of the invention can contain a gene transcription or translation enhancer, i.e. a nucleic acid which can bind to an activator, e.g., a transcription factor, to increase the gene transcription or translation level; as is known, said enhancer can be adjacent to or far from the gene on which it acts. As used in this description, the term "enhancer" includes both gene transcription enhancers and gene translation enhancers, including the regions known as IRES (Internal Ribosome Entry Site) which, as is known, are regions favoring translation initiation. If desired, said enhancer can be formed in a cassette together with a (previously defined) polynucleotide linker comprising the nucleotide sequence encoding a post-translational (auto)proteolytic cleavage site. Virtually any suitable gene transcription or translation enhancer can be used in the context of the present invention. By way of illustration, said enhancer can be the minimum SFV translation enhancer "b1" comprising the nucleotide sequence encoding the first 34 amino acids of the SFV capsid. In a particular embodiment, the viral vector of the invention comprises the polynucleotide comprising the nucleotide sequence encoding the heterologous product of interest fused at its 5' end to an enhancer, or, alternatively, to a cassette comprising said enhancer and a polynucleotide linker comprising the nucleotide sequence encoding a (previously defined) post-translational (auto)proteolytic cleavage site. In a specific embodiment, the viral vector of the invention comprises the human IGF-I precursor sequence (IGF-IB) fused at its 5' end with a cassette comprising the minimum translation enhancer ("b1") of the SFV capsid followed by the nucleotide sequence encoding the FMDV autoprotease 2A (pSFVb12A-IGF-IB, Example 10).

The RNA or DNA constructs for preparing the viral vector of the invention can be obtained by means of conventional molecular biology methods included in general laboratory manuals, for example in "Molecular cloning: a laboratory manual" (Joseph Sambrook, David W. Russel Eds. 2001, 3a ed. Cold Spring Harbor, New York) or in "Current protocols in molecular biology" (F. M. Ausubel, R. Brent, R. E. Kingston, D. D. Moore, J. A. Smith, J. G. Seidman and K. Struhl Eds, vol. 2. Greene Publishing Associates and Wiley Interscience, New York, N. Y.. Updated to September 2006.

The viral vector of the invention, derived from the noncytopathic double mutant isolated in SFV S2-9 (P718T/R649H) shows expression levels very similar to those of the wild type SFV vector.

The viral vector of the invention can be used for the *in vitro* generation of stable cell lines which can constitutively express heterologous products of interest. The use of said viral vector of the invention for the *in vitro* generation of stable cell lines which can constitutively express heterologous products of interest forms an additional aspect of the present invention.

### Stable cell line of the invention

The viral vector of the invention can be used for the *in vitro* generation of stable cell lines which can constitutively express heterologous products of interest.

Therefore, in another aspect, the invention relates to a stable cell line which can constitutively express heterologous products of interest, hereinafter stable cell line of the invention, characterized in that it is a cell line transfected with a viral vector of the invention, comprising an SFV replicon, wherein said replicon comprises (i) the nucleotide sequence encoding the SFV replicase enzyme with mutations P718T and R649H in subunit nsp2, (ii) a polynucleotide comprising a selection gene, and (iii) a polynucleotide comprising a nucleotide sequence encoding a heterologous product of interest.

As mentioned above, according to the present invention, a cell line is "stable" when the percentage of cells expressing the heterologous product of interest in passage 10 is greater than 85%, said percentage being able to be maintained or exceeded in successive or subsequent passages. Likewise, an expression is "constitutive" when the aforementioned stable cell lines can furthermore express the heterologous product at a quantitatively high level (greater than 50% with respect to that achieved 24 hours after transduction when the cells are transduced with the wild type vector).

In a particular embodiment, the stable cell line of the invention is a stable cell line transfected with a viral vector of the invention the SFV replicon of which comprises sequences SEQ ID NO: 1 and SEQ ID NO: 2.

In another particular embodiment, the stable cell line of the invention is a stable cell line transfected with a viral vector of the invention comprising:
a) a polynucleotide comprising the nucleotide sequence encoding a heterologous product of interest, the expression of which is controlled by a first SFV subgenomic promoter (SG1) ; and
b) a polynucleotide comprising a selection gene, the expression of which is controlled by a second SFV subgenomic promoter (SG2).

Said first and second SFV subgenomic promoter can be identical or different.

In another particular embodiment, the stable cell line of the invention is a stable cell line transfected with a viral vector of the invention in which the polynucleotide comprising the nucleotide sequence encoding a heterologous product of interest is located downstream of a first subgenomic promoter (SG1) overlapped in the replicase and its expression is controlled by said promoter SG1, and the polynucleotide comprising the selection gene is located downstream of a second subgenomic promoter (SG2) controlling the expression of said polynucleotide comprising the selection gene. Said subgenomic promoters SG1 and SG2 can be identical or different. The relative position between both polynucleotides can vary according to that mentioned above in relation to the viral vector of the invention.

In another particular embodiment, the stable cell line of the invention is a stable cell line transfected with a viral vector of the invention comprising, downstream of a subgenomic promoter (e.g., SG1), a construct comprising the polynucleotide comprising the selection gene and the polynucleotide comprising the nucleotide sequence encoding the heterologous product of interest, fused in phase by means of a polynucleotide linker; said linker is advantageously a polynucleotide comprising the nucleotide sequence encoding a post-translational (auto)proteolytic cleavage site. The characteristics of said linker have already been mentioned above in relation to the viral vector of the invention. In a particular embodiment, said linker comprises the nucleotide sequence encoding an (auto)protease acting in cis between the proteins resulting from the translation of the selection gene and of the nucleotide sequence encoding the heterologous product of interest, such as the nucleotide sequence encoding region 2A of the foot and mouth disease virus (FMDV) polyprotein or FMDV autoprotease 2A, or the nucleotide sequence encoding the carboxy terminal domain of the SFV capsid, etc. In another particular embodiment, said linker is a polynucleotide comprising the nucleotide sequence encoding a cleavage site for a protease acting in trans, such as the nucleotide sequence encoding a cleavage site for a viral protease, for example, the ETV protease, etc., in this case, said protease could be expressed by the cell line of the invention or, alternatively, said protease could be exogenously added. In another particular embodiment, said linker is a polynucleotide comprising the nucleotide sequence encoding a cleavage site which can be recognized by a chemical reagent, e.g., cyanogen bromide, cleaving in the methionine residues, etc., as mentioned above.

In this particular embodiment of the stable cell line of the invention in which a viral vector of the invention is used comprising, downstream of a subgenomic promoter, a construct comprising the polynucleotide comprising the selection gene and the polynucleotide comprising the nucleotide sequence encoding the heterologous product of interest, fused in phase by means of a polynucleotide linker, the subgenomic promoter (e.g., SG1) controls the expression of said construct comprising the polynucleotide comprising the selection gene and the polynucleotide comprising the nucleotide sequence encoding the heterologous product of interest, fused in phase by means of said linker, the cotranslation of both genes (selection gene and gene of interest) occurring in a single polyprotein which, after the translation, is quickly cleaved by means of rupture in the (auto)proteolytic cleavage site, producing the selection protein and the heterologous product of interest. The relative position between both polynucleotides inside the aforementioned construct can vary according to that mentioned above in relation to the viral vector of the invention, i.e., in said construct, the 3' end of the polynucleotide comprising the selection gene can be fused in phase by means of said linker to the 5' end of the polynucleotide comprising the nucleotide sequence encoding the heterologous product of interest or vice versa, the 3' end of the polynucleotide comprising the nucleotide sequence encoding the heterologous product of interest can be fused in phase by means of said linker to the 5' end of the polynucleotide comprising the selection gene. Several assays carried out by the inventors have shown that this type of viral vectors of the invention comprising, downstream of a subgenomic promoter, a construct comprising the polynucleotide comprising the selection gene and the polynucleotide comprising the nucleotide sequence encoding the heterologous product of interest, fused in phase by means of a linker comprising a DNA sequence, generate very stable cell lines maintaining a high expression of the heterologous product of interest similar to that obtained with a wild type SFV vector.

Both the characteristics of the selection gene and of the polynucleotide comprising the nucleotide sequence encoding the heterologous product of interest have been mentioned in relation to the viral vector of the invention.

In a particular embodiment, the selection gene present in the viral vector of the invention used to generate the stable cell line of the invention is a gene the expression of which confers resistance to an antibiotic, a gene which allows synthesizing an essential nutrient which is omitted in the culture medium, a gene offering a selective advantage to the transfected cells, etc., for example, the gene conferring hygromycin resistance (hph), the gene conferring neomycin resistance (neoR), the gene encoding the N-acetyltransferase puromycin enzyme (pac), the expression of which confers to the cells carrying the viral vector of the invention resistance to the puromycin antibiotic, etc.

Likewise, in a particular embodiment, the heterologous product of interest expressed by the stable cell line of the invention is a reporter protein (β-gal, etc.); or a protein, peptide or antibody (or fragment thereof) with therapeutic or diagnostic applications; or any another recombinant protein or peptide of interest; illustrative non-limiting examples of said heterologous products of interest include peptides and proteins with therapeutic applications, e.g., IGF-I, CT1, OSM, IFN, AR, GDNF, EPCR, antibodies of interest or with therapeutic or diagnostic application, etc.

In a particular embodiment, the stable cell line of the invention is a stable cell line constitutively expressing rat cardiotrophin-1 (rCT). Two types of cell lines were generated by means of using two different viral vectors of the invention. In one case, the rCT and pac expression from a viral vector of the invention containing 2 subgenomic promoters (SFV-S2-9-rCT-pac) gave rise to the generation of cells lines in which the expression remained stable for the first 5 passages, gradually being lost from passage 5 onwards (Figure 16). However, in another case, when the viral vector of the invention identified as SFV-S2-9-pac2A-rCT was used, in which the pac gene and the gene encoding rCT are fused in phase using the nucleotide sequence encoding the FMDV autoprotease 2A as a linker, very stable cell lines were obtained which maintained a high rCT expression similar to that obtained with a wild type SFV vector, for at least 10 passages in culture (Figure 17).

In another particular embodiment, the stable cell line of the invention is a stable cell line expressing insulin-like growth factor I (IGF-I). Two types of cell lines were generated by means of using two different viral vectors of the invention. In one case, the IGF-I and pac expression from a viral vector of the invention containing 2 subgenomic promoters (SFV-S2-9-IGF-pac) gave rise to the generation of cell lines in which the expression remained stable for the first 4-5 passages, gradually being lost from passage 5 onwards and decreasing 80-fold reaching passage 10 (Figure 20). However, when the viral vector of the invention identified as SFV-S2-9-pac2A-IGF was used, in which the pac gene and the gene encoding IGF-I are fused in phase using the nucleotide sequence encoding FMDV autoprotease 2A as a linker, cell lines with a higher stability were generated which maintained a high IGF-I expression similar to that obtained with a wild type SFV vector, with only an approximate 4-fold decrease in 10 passages in culture (Figure 20). The latter vector (SFV-S2-9-pac2A-IGF) further showed IGF-I expression levels in early passages which were approximately 2 times greater than those observed with vector SFV-S2-9-IGF-pac.

The stable cell lines of the invention can be obtained by means of conventional molecular biology methods, for example, by means of transfecting suitable cells or cell lines with the viral vector of the invention, etc., included in general laboratory manuals, for example, in "Molecular cloning: a laboratory manual" (Joseph Sambrook, David W. Russel Eds. 2001, 3rd ed. Cold Spring Harbor, New York) or in "current protocols in molecular biology" (F. M. Ausubel, R. Brent, R. E. Kingston, D. D. Moore, J. A. Smith, J. G. Seidman and K. Struhl Eds, vol. 2. Greene Publishing Associates and Wiley Interscience, New York, N. Y. Updated to September 2006.

The cell to be transfected can be virtually any eukaryotic cell or cell line allowing the replication of the viral vector of the invention. Said cell or cell line can be a line from a mammal. In a particular embodiment, said cell or cell line to be transfected is a cell line derived from hamster kidney fibroblasts, the BHK-21 cell line, for example.

The transfection of the cells is carried out by means of any conventional physical or chemical process which allows introducing the viral vector of the invention into the cells, by electroporation or by conjugation of the genetic material with cationic lipids, for example. These processes will be useful for trasfecting the viral vector of the invention either as RNA or as DNA. In a particular embodiment, the transfection is carried out by electroporation ("Current protocols in molecular biology"; Ausubel FM et al.; citado supra).

### Method for generating in vitro stable cell lines of the invention

In another additional aspect, the invention relates to a method for generating *in vitro* of a stable cell line of the invention, with the capacity to constitutively express heterologous products of interest, hereinafter method for generating stable cell lines of the invention, which comprises:
I. transfecting cells with a viral vector of the invention, comprising an SFV replicon, wherein said replicon comprises (i) the nucleotide sequence encoding the SFV replicase enzyme with mutations P718T and R649H in subunit nsp2, (ii) a polynucleotide comprising a selection gene, and (iii) a polynucleotide comprising a nucleotide sequence encoding a heterologous product of interest;
II. selecting the stable cells generated in step I; and
III. growing and maintaining the stable cells.

As mentioned above, the cell to be transfected can be virtually any eukaryotic cell or cell line allowing the replication of the viral vector of the invention. By way of a non-limiting illustration, said cell or cell line to be transfected can be a line from a mammal. In a particular embodiment, said cell or cell line to be transfected is a cell line derived from hamster kidney fibroblasts, the BHK-21 cell line, for example.

The characteristics of the viral vector of the invention have already been mentioned previously. The viral vector of the invention can be used in RNA form or, alternatively, in DNA form. When it is used in RNA form, the viral vector of the invention is completed with the addition of a cap structure at its 5' end. When it is used in DNA form, the complete vector includes a functional promoter in eukaryotic cells, a cytomegalovirus (CMV) promoter, for example, the SFV replicon sequence in any of the embodiments defined previously in relation to the viral vector of the invention, and a transcription termination signal sequence, a signal sequence derived from SV40, for example.

The cells can be transfected by any conventional physical or chemical process which allows introducing the viral vector of the invention into the cells, by electroporation or by conjugation of the genetic material with cationic lipids, for example. These processes will be useful for transfecting the viral vector of the invention either as RNA or as DNA. In a particular embodiment, the transfection is carried out by electroporation ("Current protocols in molecular biology"; Ausubel FM et al.; mentioned above).

Stable cells will be selected in a different manner according to the selection gene incorporated in the viral vector of the invention whereby the cells are transfected. As explained above, the expression of the selection gene confers to the transfected cells an advantage allowing their selection. It will be enough to subject the cells to selective conditions for the transfected cells. When the pac gene is used as the selection gene, its expression transfected in the cells makes them puromycin-resistant. In this case, it will be enough to add puromycin to the culture medium to eliminate the non-transfected cells or the cells which have lost the viral vector of the invention.

The stable cells are grown an maintained in conventional culture mediums and conditions, according to the transfected cell type, and maintaining them under the selective stimukus for transfected cells (in the presence of puromycin, for example). When the cells are BHK-21, they are grown in already described conditions ("Current protocols in molecular biology"; Ausubel FM et al.; mentioned above).

### Method for producing in vitro heterologous products of interest

The stable cell line of the invention can be used for producing *in vitro* heterologous products of interest. As a result, the use of said stable cell line of the invention for the *in vitro* production of heterologous products of interest forms and additional aspect of the present invention.

Therefore, in another aspect, the invention relates to a method for producing *in vitro* heterologous products of interest which comprises culturing a stable cell line of the invention under conditions which allow the expression of the heterologous product of interest contained in the viral vector of the invention used to generate said stable cell line of the invention.

More specifically, the method for producing *in vitro* heterologous products of interest comprises:
I. transfecting cells with a viral vector of the invention comprising an SFV replicon, wherein said replicon comprises (i) the nucleotide sequence encoding the SFV replicase enzyme with mutations P718T and R649H in subunit nsp2, (ii) a polynucleotide comprising a selection gene, and (iii) a polynucleotide comprising a nucleotide sequence encoding a heterologous product of interest;
II. selecting the stable cells generated in step I (stable cell lines of the invention);
III. growing and maintaining said stable cells (cell lines of the invention); and, if desired,
IV. extracting the heterologous product of interest.

Steps I, II and III actually correspond to the generation of the stable cell line of the invention constitutively expressing the heterologous product of interest. In this case, the cell will be transfected with a viral vector of the invention including a polynucleotide comprising the nucleotide sequence encoding the heterologous product of interest which is to be produced. As indicated above, said polynucleotide comprising the nucleotide sequence encoding the heterologous product of interest is inserted in the viral vector of the invention in a position such that its expression is controlled by an SFV subgenomic promoter.

As mentioned above, the heterologous product of interest can be any protein or peptide of interest, e.g., a reporter protein (β-gal, etc.); or a protein, peptide or antibody (or fragment thereof) with therapeutic or diagnostic applications; or any another recombinant protein or peptide of interest. Illustrative non-limiting examples of heterologous products of interest include peptides and proteins with therapeutic applications, e.g., insulin-like growth factor I (IGF-I), cardiotrophin-1 (CT1), oncostatin M (OSM), interferon alpha (e.g, IFNa5), amphiregulin (AR), glial cell-derived neurotrophic factor (GDNF), endothelial cell protein C/activated protein C receptor (EPCR), antibodies of interest or with therapeutic or diagnostic application, etc.

The steps for generating the stable cell line of the invention will be carried out as described previously in the method for generating stable cell lines of the invention.

The heterologous product of interest can occasionally be used as it is in the culture medium, i.e., without being isolated or purified; however, it can occasionally be advantageous to extract (isolate) and optionally purify the heterologous product of interest. In this case, the extraction of the heterologous product of interest can be carried out from a cell lysate when the heterologous product of interest is intracellular (it cannot be secreted to the medium), or from the supernatant of the stable cells when the heterologous products of interest can be secreted to the surrounding medium.

Any conventional process for extracting and purifying peptides and proteins that is suitable for the specific heterologous product of interest which is to be produced, depending on the nature of said product, can be used for the extraction. Illustrative non-limiting examples of such processes include processes based on the separation by size and/or electric charge (precipitation in ammonium sulfate, gel filtration, ultracentrifugation, electrophoresis, electrofocusing, etc.), by immunopurification (affinity column, immunoprecipitation, etc.), separation by binding to a specific ligand, etc.

The objective of the following examples is to illustrate the invention without intending to limit it.

### EXAMPLES OF THE INVENTION

### Materials and methods

### Plasmid Constructions

Plasmids pSFV-1 and pSFV3-LacZ, kindly provided by Dr. P. Liljeström (Karolinska Institute, Stockholm) (18) have previously been described. To construct plasmids pSFV-S2 and pSFV-S3, a 3.5 kb fragment containing the area of nsp2 to be mutagenized was extracted from pSFV-1 with SacI/XhoI and was sub-cloned into plasmid pBluescript SK (Stratagene, La Jolla, USA) digested with the same enzymes, which generated plasmid Blu-nsp2. Mutations P718T and P718F were introduced in nsp2 in this plasmid by means of crossover PCR. External primers SF3669-VS (SEQ ID NO: 3) and SF4096-RS (SEQ ID NO: 4) were sequentially combined with the oligonucleotide pairs designed to introduce the respective mutations. For S2, the internal oligonucleotide pair used was mutS2-VS (SEQ ID NO: 5) and mutS2-RS (SEQ ID NO: 6) which overlap in 25 nucleotides and in which the codon CCC encoding Pro 718 in nsp2 has been mutated to ACG encoding Thr (underlined). For S3, the internal oligonucleotides used were mutS3-RS (SEQ ID NO: 7) and mutS3-RS (SEQ ID NO: 8), which overlap in 25 nucleotides and in which the codon CCC encoding Pro 718 of nsp2 has been mutated to TTT encoding Phe (underlined). Crossover PCR was carried out with Pfu and produced 430 bp DNA fragments which were digested with EagI/NarI and cloned into Blu-nsp2 after partial digestion with EagI and complete digestion with NarI, which generated plasmids Blu-nsp2-S2 or Blu-nsp2-S3, respectively. The presence of mutations S2 or S3 was confirmed in these plasmids by sequencing. Finally, a SacI/XhoI 3.5 kb fragment containing mutated nsp2 was extracted from Blu-nsp2-S2 or of Blu-nsp2-S3 and was sub-cloned into pSFV-1 digested with the same enzymes, which generated plasmids pSFV-S2 and pSFV-S3, respectively. In order to clone LacZ into these plasmids, a 3.86 kb DNA fragment containing this gene was extracted with XbaI/XmaI of pSFV3-LacZ and was cloned into pSFV-S2 or pSFV-S3 digested with the same enzymes, which generated plasmids pSFV-S2-LacZ and pSFV-S3-LacZ, respectively.

In order to generate plasmid pSFV-pac, a 0.95 kb DNA fragment containing the puromycin N-acetyltransferase (pac) gene was extracted from plasmid pBSpac (5) (kindly provided by Dr. J. Ortin, CNB, Madrid, Spain) by means of digestion with NotI and HpaI. After blunting the NotI end with Klenow, this fragment was cloned into pSFV-1 digested with SmaI, which generated pSFV-pac. Plasmid pSFV-S2-pac was generated upon substituting the 3.5 kb SacI/XhoI fragment containing most of nsp2 in pSFV-pac with the same fragment obtained from Blu-nsp2-S2, containing mutation S2.

Plasmid pSFV-S2-9-LacZ was generated upon substituting the 3.2 kb RsrII/XhoI fragment in pSFV3-LacZ with the same fragment obtained from pSFV-S2-9-pac covering the area of the replicase containing mutations P718T and R649H of nsp2. In order to generate the double vector pSFV-S2-9-LacZ-pac, a 1.9 kb cassette containing the SFV subgenomic promoter followed by the pac gene was extracted from pSFV-pac with MscI/SpeI, and was cloned into pSFV-S2-9-LacZ digested with SmaI/SpeI.

In order to generate pSFV-RHR-pac, first mutation R649H of nsp2 was introduced in Blu-nsp2 by PCR. Briefly, a 1.67 kb PCR fragment was obtained with the oligonucleotides SF1947-VS (SEQ ID NO: 9) and SF3623-S29-RS (SEQ ID NO: 10) with Pfu using pSFV-1 as a mold. In the latter oligonucleotide codon CGC has been substituted with CAC, which leads to mutation R649H in nsp2 (underlined; observe that the primer contains the reverse sequence). The sequence of this oligonucleotide comprises the NarI site of nsp2 next to mutation R649H (indicated in italics). The PCR fragment was digested with BstEII/NarI, which gave rise to a 1.36 kb DNA fragment which was cloned into Blu-nsp2 digested with the same enzymes, which thus generated Blu-nsp2-RHR. Finally, a 3.5 kb SacI/XhoI fragment containing mutated nsp2 was extracted from Blu-nsp2-RHR and was subcloned into pSFV-pac digested with the same enzymes, which generated plasmid pSFV-RHR-pac.

### RNA transcription and transfection

The purified plasmid DNA were made linear by means of digestion with SpeI and were transcribed in the presence of a cap analogue (New England Biolabs, USA) using SP6 polymerase (Amersham-Pharmacia). The RNAs synthesized *in vitro* were transfected in BHK-21 cells by means of electroporation as previously described (17).

### Packaging of replicons

The packaging of the SFV recombinant RNA in viral particles (vp) was carried out by means of the coelectroporation of BHK-21 cells with the recombinant RNA and with both auxiliary SFV helper-S2 and helper-C-S219A RNAs as is described (29). The SFV particles carrying LacZ as an indicator gene were titrated by X-gal staining of the infected BHK-21 cells with serial dilutions of the virus. For the SFV particles carrying the pac gene, titration was carried out by means of indirect immunofluorescence of the BHK-21 cells infected with a polyclonal rabbit anti-serum specific against SFV nsp2 as a primary antibody (E. Casales, results not yet published).

### Selection and passages of the puromycin-resistant cell lines

A 1 mg/ml solution of puromycin (Sigma, St. Louis, USA) in MEM was prepared, filtered, aliquoted and stored at -20°C. After transfection of the BHK cells with the SFV recombinant RNAs carrying the pac gene, the cells were left to recover for 24 hours before adding puromycin at 5 µg/ml. In order to select the puromycin-resistant cells, the medium was replaced every 2 to 3 days with a new medium containing puromycin. After the selection, the passages of the cells were always carried out in the presence of puromycin at the indicated concentración. In the case of cells transfected with SFV-S2-pac, individual loci were cloned, expanded and stored frozen in liquid nitrogen. The medium used to grow the cells was BHK-21 Glasgow MEM (Invitrogene, USA) supplemented with 5% bovine fetal serum, 10% phosphate tryptose medium (Invitrogene, USA), 2 mM glutamine, 20 mM HEPES, streptomycin at 100 µg/ml and penicillin at 100 IU/ml (complete BHK medium).

### Mapping of the adaptive mutations

The total cellular RNA of the puromycin-resistant cells obtained after transfection with the SFV-S2-pac RNA was isolated with the RNeasy kit (Qiagen, Germany). 5 µg of RNA of each clone were used to synthesize cDNA with M-MLV reverse transcriptase (Promega, Madison, USA) using a minus-sense oligonucleotide complementary to SFV nucleotides 4977 to 5010 (SEQ ID NO: 11) as a primer. After reverse transcription, the cDNAs were amplified by means of 30 cycles of PCR with Taq Plus Long (Stratagene, La Jolla, USA), with the same minus-sense primer and a plus-sense oligonucleotide complementary to SFV nucleotides 1040 to 1074 (SEQ ID NO: 12). The resulting 3971 bp fragment was digested with BcII (1106) and Bsu36I (4916) and was cloned into the corresponding pSFV-pac sites. The plasmid DNA derived from each individual clone was made linear and was used as a mold to synthesize RNA *in vitro,* which was subsequently transfected into BHK-21 cells in order to determine their capacity to confer resistance to puromycin. When the subregion comprised between positions 1106 to 4916 was able to allow cell survival and division in the presence of puromycin, as was the case with clone S2-9, it was completely sequenced from two independent plasmid clones.

### RNA analysis

The total RNA was extracted from the transfected cells or from certain cell lines using an RNeasy mini-kit (Qiagen, Germany) and was analyzed by means of Northern blot. 3 µg of total RNA were fractioned by size in 1.2% agarose gel with formaldehyde, were transferred to a nitrocellulose membrane (Hybond-N+, Amersham) and hybridized with a ³²P-labelled oligonucleotide specific for the SFV subgenomic promoter (SEQ ID NO: 13). The relative amounts of the genomic and subgenomic SFV RNAs were determined using a PhosphorImager (Cyclone, Packard, USA) and Optiquant software (version 4.0, Packard).

### Analysis of the expression and the replicase processing

For the *in vitro* translation experiments, the SFV RNAs were first transcribed *in vitro* as previously described, were purified with the RNeasy kit (Qiagen, Germany), mixed with rabbit reticulocyte lysates treated with nuclease (Promega) in the presence of a mixture of [³⁵S]-methionine and [³⁵S]-cysteine (Amersham, USA) and were incubated for 90 minutes at 30°C according to the manufacturer's instructions. Each translation reaction contained 2.3 µg of purified transcribed RNA. The translation reactions ended upon adding the Laemmli loading buffer and were analyzed by SDS-PAGE in 8% polyacrylamide gels followed by autoradiography. Lysates were obtained for the immunoblot analysis experiments from the BHK-21 cells transfected with the SFV vectors by means of incubation in a buffer containing 1% Igepal (Sigma, USA), 50 mM Tris HCl, pH 7.6, 150 mM NaCl, 2 mM EDTA and PMSF at 1 µg/ml (Sigma, St. Louis, USA), were clarified by centrifugation for 6 minutes at 6000 rpm in a refrigerated microcentrifuge and were quantified by means of Bradford analysis. The lysates were analyzed by means of SDS-PAGE in 8% polyacrylamide gels, were transferred to a nitrocellulose membrane and incubated with polyclonal rabbit anti-serums specific against SFV nsp3 and nsp2 (E. Casales, results not yet published) or actin (Sigma, St. Louis, USA) as primary antibodies, respectively. A sheep antiserum specific for rabbit immunoglobulins conjugated with HRP was used as a secondary antibody. The proteins were viewed using Western Lightning Chemiluminiscence Reagent Plus (Perkin Elmer Life Sciences, USA), according to the manufacturer's instructions. For the nuclear localization studies, indirect immunofluorescence of the transfected cells was carried out using as a primary antibody an Acm specific against cytoplasmic SFV nsp2 (kindly provided by W. Bodemer) or an Acm specific against the nuclear SFV nsp2 (kindly provided by Dr. L. Kaariainen) (16).

### Analysis of the β-gal expression

The transfected cells were lysated as described above and total activity of the β-gal present in the lysate was measured as described (17), or they were stained at the indicated times with X-gal. The amount of the protein produced by each cell was obtained by dividing the mean amount of β-gal detected per plate by the mean amount of the transfected cells in each plate.

### EXAMPLE 1

### Construction and characterization of SFV vectors carrying noncytopathic mutations derived from SIN

A possible approach for generating new noncytopathic alphavirus vectors is to introduce the previously described mutations into other alphaviruses, taking advantage of the sequence homology shared by the different viruses of this genus. According to this approach, the effect of the noncytopathic mutations described in the SIN virus in an expression vector derived from SFV was evaluated. It had previously been demonstrated that the mutations that affected residue 726 of subunit nsp2 of the SIN Rep considerably reduce the cytopathogenicity of this virus, a fact which was correlated with a lower RNA replication level in the mutants (7, 22). Two mutations that affected this residue, P726T and P726F, which had been described as partially cytopathic and noncytopathic for SIN, respectively, were introduced into the homologous residue of nsp2 in vector pSFV-1, which generated mutants pSFV-S2 and pSFV-S3, carrying mutations P718T or P718F in nsp2, respectively. In order to evaluate the cytopathogenicity of these new vectors, the LacZ reporter gene was then cloned from the viral subgenomic promoter, which generated vectors pSFV-S2-LacZ and pSFV-S3-LacZ. The RNA was synthesized *in vitro* for each of these plasmids and was electroporated in BHK-21 cells, which were cultured and in which β-gal expression was analyzed by means of X-gal staining at different times after transfection (Figure 1). Unlike the cells electroporated with the control SFV-LacZ RNA in which, 24 hours after transfection, more than 95% of the cells expressed β-gal and showed a cytopathic phenotype, only a small percentage of the cells transfected with the SFV-S2-LacZ or SFV-S3-LacZ RNAs expressed β-gal 24 hours after transfection, without showing a cytopathic morphology. In the case of SFV-S2-LacZ, the number of positive cells increased over time, being able to form large colonies of cells expressing β-gal 5 days after electroporation. This effect was considerably less in SFV-S3-LacZ, with which blue colonies were never observed on day 5 after X-gal staining. This data suggests that mutations P718T and P718F could be blocking viral replication or the transgene expression, but that they give rise to noncytopathic variants with relatively high frequency that could be the result of secondary adaptive mutations during *in vitro* transcription or replication of SFV.

### EXAMPLE 2

### Selection and mapping of the replicons derived from SFV-S2 containing new adaptive mutations

In order to select the cell populations containing noncytopathic SFV replicons, the LacZ reporter gene was substituted in plasmid pSFV-S2-LacZ with the gene encoding the puromycin N-acetyltransferase (pac) dominant selection marker conferring resistance to puromycin, which generated plasmid pSFV-S2-pac. Using this plasmid as a mold, RNA was synthesized *in vitro* and electroporated in BHK-21 cells. Puromycin at 5 µg/ml was added 24 hours after electroporation, which led to the selection of colonies resistant to the drug which subsequently were expanded. In order to identify the possible adaptive mutations in the replicons present in the selected cells, total RNA was extracted from individual clones, and a 3.9 kb fragment of the SFV replicase comprising the entire nsp2 sequence was amplified by RT-PCR with specific primers. The nsp2 region of the replicase was amplified because most of the noncytopathic mutations described in the alphavirus had been mapped in this region. The cDNA fragment rescued from each clone was subcloned into the original plasmid pSFV-pac substituting the wild type nsp2 sequence. To check if noncytopathic mutations had been rescued, RNA was synthesized *in vitro* of each of the new plasmids and was used to electroporate BHK-21 cells. 24 hours after transfection, puromycin at 5 µg/ml was added and the cells were stained with methyl violet at different times in order to compare their growth with that observed in the cells transfected with the initial mutant SFV-S2-pac or with the original SFV-pac. One of the clones, the SFV-S2-9-pac, showed a clear noncytopathic phenotype, since it conferred puromycin resistance to most of the transfected cells (Figure 2). Other tested clones showed a similar pattern to that of SFV-S2 and were not further analyzed in this study. The cells transfected with the original. SFV-pac showed a clear cytopathic effect and most of them died before day 4. Sequencing of the nsp2 fragment of pSFV-S2-9-pac showed that in addition to the original mutation P718T, there was a second adaptive mutation in position 649 of nsp2 in which Arg had been substituted with Hys. This new mutation affects the nuclear localization signal ⁶⁴⁸RRR (27) of SFV nsp2 which, in SFV-S2-9, changes to ⁶⁴⁸RHR.

### EXAMPLE 3

### Effect of mutation R649H on cytopathogenicity and on the nuclear localization of nsp2

In order to check the effect of mutation R649H alone on the cytopathogenicity of SFV, this mutation was introduced in plasmid pSFV-pac, thus generating plasmid pSFV-RHR-pac. The RNA of this plasmid was transcribed and electroporated in BHK-21 cells, which were left to recover for 24 hours. Puromycin at 5 µg/ml was then added and the growth of the cells was analyzed at different times by staining the cells with methyl violet as described above (Figure 2, right column). SFV-RHR-pac showed a very similar pattern to the one observed for the original vector SFV-pac, inducing a strong and early cytopathic effect that led to the death of most of the cells before day 4. These results suggest that the absence of cytopathogenicity of vector SFV-S2-9 was due to the combination of mutations P718T and R649H. In order to check the effect of mutation R649H on the nuclear localization of nsp2, BHK-21 cells were transfected with the RNA of the vectors that contained this mutation (SFV-S2-9-pac and SFV-RHR-pac) or with vectors that did not contain them (SFV-pac and SFV-S2-pac) and were analyzed by means of immunofluorescence with monoclonal antibodies specific for the nuclear form or for the cytoplasmic form of SFV nsp2, respectively (Figure 3). In all cases, nsp2 was detected both in the nucleus and in the cytoplasm of the transfected cells, which suggest that mutation RHR did not affect transport to the nucleus of this protein.

### EXAMPLE 4

### Replication of RNA in cells transfected with SFV-S2-9-pac

In order to determine the RNA replication level of the double noncytopathic SFV mutant, BHK-21 cells were electroporated with SFV-S2-9-pac RNA, total RNA was extracted 24, 48 and 72 hours after electroporation and was analyzed by means of Northern blot with a ³²P-labelled oligonucleotide specific for the sequence of the SFV subgenomic promoter, which is present both in the genomic RNA and in subgenomic RNA (Figure 4, lanes 2 to 4). The amount of the genomic and subgenomic SFV-S2-9-pac RNAs, as well as the proportion between them, was compared with that obtained in cells transfected with SFV-pac RNA 24 hours after electroporation (Figure 4, lane 5). The genomic and subgenomic SFV-S2-9-pac RNAs increased over time and seemed to reach a peak 48 hours after electroporation. Similar RNA levels were observed in a puromycin-resistant BHK cell line obtained with vector SFV-S2-9-pac (Figure 4, lane 1). In all cases, the SFV-S2-9 RNAs were produced in a much lower amount than that obtained in cells transfected with the original SFV-pac RNA (note that lane 5 was exposed 72 times more than lanes 1 to 4). The subgenomic/genomic RNA ratios observed in the cells transfected with SFV-S2-9-pac and selected with puromycin was approximately 1.5 times greater than that observed in cells transfected with SFV-pac (Figure 4, numbers in the lower part). However, in the cells transfected with SFV-S2-9-pac not selected, this ratio was considerably greater, especially 24 hours after transfection, but decreased over time, which indicated that the genomic RNA synthesis in the double mutant could be delayed in relation to that of the subgenomic RNA. Two additional bands of RNA with an intermediate size between the genomic and subgenomic RNAs were also detected in the cells transfected with SFV-S2-9-pac (Figure 4, lanes 1 to 4). These bands were not characterized but they could correspond to defective-interfering RNAs that may appear during replication of the double SFV mutant. Finally, in order to determine if the low replication levels of the SFV-S2-9-pac RNA could be recovered with a wild type replicase supplied in trans, the BHK-21 cells were electroporated simultaneously with the SFV-S2-9-pac and of SFV-LacZ RNAs (wild vector). After 24 hours, the total RNA was extracted from the coelectroporated cells and was analyzed by means of Northern blot (Figure 4, lane 7). The RNAs of the cells electroporated with the original SFV-pac (Figure 4, lane 5) and SFV-LacZ (Figure 4, lane 6) were analyzed in the same gel and were used as molecular size markers for each of the genomic and subgenomic RNAs. The genomic and subgenomic SFV-LacZ and SFV-S2-9-pac RNAs were readily detected in the cells coelectroporated after exposing the gel for 1 hour, which indicated that the wild type replicase was able to at least partially recover the replication of the double mutant. However, although the amount of both RNAs subgenomic was very similar, the genomic SFV-LacZ RNA was produced at levels approximately 2-3 times greater than that of mutant SFV-S2-9-pac.

### EXAMPLE 5

### Replicase processing in mutant SFV-S2-9

The smaller amount of RNA detected in the cells transfected with the SFV-S2-9-pac replicon could be due to an alteration in replicase processing. The SFV replicase is synthesized as polyprotein nsp1234 which is subsequently cleaved into its mature monomeric components by means of the protease activity present in the domain of the carboxyl end of nsp2 (30), in which mutations P718T and R649H have been mapped. In order to examine the effect of these mutations on replicase processing, the SFV-S2-9-pac, SFV-RHR-pac and SFV-S2-pac RNAs synthesized *in vitro* were translated by means of incubation with rabbit reticulocyte lysates in the presence of [³⁵S]-methionine and [³⁵S]-cysteine. The replicase processing pattern in these mutants was compared with that obtained for the original SFV-pac RNA by means of SDS-PAGE followed by autoradiography (Figure 5A). No difference was observed in the replicase processing between the three analyzed mutants and the wild type replicase, which gave rise to a similar accumulation of the nsp monomers in all cases. In order to determine if the replicase processing was affected in vivo, the lysates of a puromycin-resistant BHK-21 cell line carrying vector SFV-S2-9-pac or of BHK-21 cells transfected with original SFV-pac RNA or with SFV-RHR-pac RNA obtained 24 hours after electroporation were analyzed by means of immunoblotting with a rabbit antiserum specific against nsp2 (Figure 5B, upper panel) or against nsp3 (Figure 5B, central panel) as primary antibodies, respectively. In both cases, the samples derived from SFV-pac and from SFV-RHR-pac showed a very similar pattern as they expressed comparable amounts of the nsp2 and nsp3 monomers. The amount of nsp2 and nsp3 in the puromycin-resistant BHK-SFV-S2-9-pac cell line was approximately 1.5 times lower, which could be due to the lower replication of this vector. Even though only insignificant amounts of high molecular weight products were detected in the analyzed samples, differential bands could be observed between SFV-S2-9 and the two other vectors with the antiserum against nsp2, which could indicate a small alteration of the *in vivo* S2-9 replicase processing.

### EXAMPLE 6

### Expression of the transgene and packaging of the SFV-S2-9 replicon

In order to determine the expression level of the heterologous gene in mutant SFV-S2-9, the Lac-Z reporter gene after the subgenomic promoter of SFV was cloned into this vector, which generated plasmid pSFV-S2-9-Lac-Z. RNA was synthesized *in vitro* from this plasmid and was used to electroporate BHK-21 cells, in which β-gal expression was analyzed at different times after electroporation. Cells were also transfected with RNA of the original vector SFV-LacZ, but they were only analyzed 24 hours after electroporation due to the cytopathic effect induced by this vector. A similar β-gal expression level was observed in the cells transfected with SFV-S2-9-LacZ and SFV-LacZ 24 hours after transfection, this being approximately 15 pg/cell (Figure 6). This expression level increased over time in the cells transfected with SFV-S2-9-LacZ, stabilizing at approximately 30 pg of β-gal/cell 48 hours after transfection. In order to determine the packaging efficiency of double mutant SFV-S2-9, BHK-21 cells with SFV-S2-9-LacZ RNA and the auxiliary SFV-helper-S2 and SFV helper-C-S219A RNAs were coelectroporated as described (29). The supernatants of the cells were collected 24 hours after electroporation and were titrated on monolayers of BHK-21 cells by means of X-gal staining. The SFV-S2-9-LacZ RNA was rather inefficiently packaged, which provided titers of 7 x 10³ pv/ml, which were much lower than those obtained the original SFV-LacZ (5 x 10⁹ pv/ml). In order to compare if the presence of the wild type replicase could increase the packaging efficiency of double mutant S2-9, the BHK-21 cells with the RNAs of SFV-S2-9-LacZ, SFV-pac and both SFV auxiliary RNAs were coelectroporated. In this case, it was observed the original vector SFV was able to moderately increase the production of SFV-S2-9-LacZ particles, which reached a titer of 1 x 10⁵ pv/ml 24 hours after electroporation, which was determined by means of X-gal staining of the infected BHK cells. The packaging of the original SFV-pac RNA decreased approximately 5-fold in the cells in which this vector was coelectroporated with SFV-S2-9-LacZ (2.6 x 10⁸ pv/ml), in comparison with the titers obtained when the SFV-pac was packaged alone (1.2 x 10⁹ pv/ml), which was determined by means of indirect immunofluorescence of the infected cells using a polyclonal rabbit antiserum specific against SFV nsp2 as a primary antibody. In order to determine the effect that each of the two individual mutations present in SFV-S2-9 has on the packaging, the BHK-21 cells with both auxiliary SFV RNAs and with the SFV-RHR-pac or SFV-S2-pac RNAs were coelectroporated, and the viral particles were titrated as described above for SFV-pac. SFV-RHR-pac was packaged with high titers (6.5 x 10⁹ pv/ml) whereas SFV-S2-pac showed a very low packaging efficiency (2 x 10⁴ pv/ml).

### EXAMPLE 7

### Production of a stable cell line expressing LacZ using vector SFV-S2-9

As has been shown in this study, vector SFV-S2-9-pac can be used to select the cells which can maintain the replicon in the presence of puromycin. To study if it was possible to use this type of vector for generating stable cell lines expressing other transgenes, the LacZ reporter gene was introduced into pSFV-S2-9-pac, which generated plasmid pSFV-S2-9-LacZ-pac in which the pac and LacZ genes are located downstream of independent subgenomic promoters. The RNA of this plasmid was synthesized *in vitro* and was electroporated in the BHK-21 cells. 24 hours after the electroporation, puromycin at 5 µg/ml was added and when the selected cells reached confluence, ten passages were carried out in the presence of the antibiotic for a period of 30 days. The percentage of cells expressing β-gal, determined in each passage by means of X-gal staining, varied between 70% and 90% according to the passage, but was above 85% in passage 10, which indicated a high stability of the transgene expression in the presence of selection (Figure 7). When the selected cells were passaged without puromycin, the percentage of the cells expressing β-gal decreased very quickly, being under 5% after 3 passages. The β-gal expression level in the selected cells with puromycin was determined after 6 and 8 passages, reaching 13 and 18 pg/cell, respectively. These values are very similar to those obtained in the cells transfected with the original vector SFV-LacZ 24 hours after transfection, which indicated that the cell lines generated with vector SFV-S2-9 could be used to express recombinant proteins large amounts.

### EXAMPLE 8

### Evaluation of other previously defined noncytopathic SFV mutants

The mutations which had been previously defined as noncytopathic for SFV were introduced into the gene of subunit nsp2 of the SFV replicase, in plasmid pSFV-1, containing the vector sequence. These mutations included:
- L10T (change of TTG for ACC): mutant SF2A (22).
- L713P (change of CTA for CCT): mutant SF2C (22).

Furthermore, plasmid pSFV-PD containing the SFV vector sequence with mutations S259P and R650D in nsp2 was kindly provided by Dr. K. Lundström (21).

Plasmids pSFV-SF2A, pSFV-SF2C and pSFV-PD were thus generated (or obtained), into which the LacZ gene was cloned under the control of the viral subgenomic promoter, plasmids: pSFV-SF2A-LacZ, pSFV-SF2C-LacZ and pSFV-PD-LacZ, respectively, being obtained.

These plasmids were used to synthesize RNA *in vitro,* which was electroporated in BHK-21 cells. After the electroporation, the cells were distributed in different plates which were fixed and stained with X-gal at different times after transfection. The X-gal staining causes a blue color in the cells which are expressing β-gal from the LacZ gene, which allows detecting the number of cells carrying the vector and which have survived at different times as well as analyzing the morphology thereof, which is a cytopathogenicity indicator. In this experiment, cells electroporated with SFV-LacZ RNA, which is cytopathic for BHK cells, were used as a negative control. Likewise SFV-S2-LacZ RNA, carrying mutation P718T in nsp2 and giving rise to a noncytopathic phenotype in a certain percentage of the cells, was used as a positive control.

As can be observed in Figures 10 and 12, mutants SFV-SF2A and SFV-PD shows a phenotype that was very similar to that of the wild SFV vector, inducing a strong cytopathic effect in the transfected cells, which was translated into the appearance of a number of apoptotic bodies from day 3 onwards and an almost total absence of expression after long times (7 days). The data relating to the cytopathic nature of the SFV-PD vector are further supported by results published subsequently by K. Lundström himself in which it is shown how the β-gal or GFP expression in BHK cells infected with a SFV-PD vector carrying LacZ or GFP as a reporter gene reaches a maximum after 48 hours to later decrease drastically in the next 3-4 days (20, see Figure 2 of this reference). Mutant SFV-SF2C showed a somewhat less cytopathic phenotype, but many apoptotic bodies appear on day 3, and although apparently there are high expression levels on day 4, this disappears almost completely on day 7, indicating that this mutant probably allows somewhat more prolonged expressions than the wild vector, but without stopping to be cytopathic. Vector SFV-S2 is the only vector that does not give rise to the appearance of apoptotic bodies and which allows maintaining the expression for at least 7 days, giving rise to the appearance of colonies. Vector SFV-S2-9 carrying mutations P718T and R649H was selected from one of the colonies obtained with SFV-S2 when the LacZ gene was substituted with the pac gene in said vector. Figure 14 includes photos of cells transfected with vector SFV-S2-9-LacZ RNA and stained at different times with X-gal. Vector S2-9 did not produce a cytopathic effect in the transfected cells, which were able to divide.

### EXAMPLE 9

### Obtaining stable cell lines producing cardiotrophin 1 using vector SFV-S2-9

Plasmids were generated by introducing the rat cardiotrophin (rCT) gene in pSFV-S2-9-pac in two different environments and were used to generate cell lines expressing rCT. Both the rCT expression and the stability of the rCT expression were then analyzed for the purpose of identifying stable cell lines expressing rCT.

### 9.1 Plasmid Construction

For the purpose of generating cell lines expressing cardiotrophin, plasmids based on SFV-S2-9-pac were generated by introducing the rat cardiotrophin (rCT) gene, according to two different embodiments:
- plasmid pSFV-S2-9-rCT-pac, in which the pac and rCT genes are placed downstream of independent subgenomic promoters (Figure 15A); and
- plasmid pSFV-S2-9-pac2A-rCT, constructed according to the general structure described in Figure 8B, containing the pac gene and the rCT gene fused downstream of a single subgenomic promoter (Figure 15A); in this embodiment, the FMDV autoprotease 2A sequence (SEQ ID NO:14) (35) was introduced between the two genes to allow the release of the cardiotrophin from the fusion protein (Figure 15A).

To construct plasmid pSFV-S2-9-rCT-pac, the cloning vector pSFV-S2-9-mcs-pac, containing the SFV-S2-9 replicase, a first subgenomic promoter followed by a multiple cloning site and second subgenomic promoter followed by the pac gene were generated previously.

Briefly, oligonucleotides SEQ ID NO: 15 and SEQ ID NO: 16 were hybridized, generating a synthetic DNA fragment with BamHI-compatible protuberant 5' ends, and the sequence of which contains a multiple cloning site with targets for enzymes AvrII, ApaI, NruI and BstBI, three translation termination codons in the three possible reading stages and the SFV subgenomic promoter sequence. This fragment was cloned into the BamH I site of pSFV-S2-9-pac, plasmid pSFV-S2-9-mcs-pac being generated.

In a second step, a 645 bp PCR fragment containing the rat cardiotrophin gene was synthesized from plasmid pRSET-rCT (36), using oligonucleotides SEQ ID NO: 17 and SEQ ID NO: 18 hybridizing with the start and end of the rCT gene respectively and containing BamH I sites at the ends to make the cloning easier. The PCR fragment was digested with BamH I and was cloned into the BamH I site of pSFV-S2-9-mcs-pac, plasmid pSFV-S2-9-rCT-pac being obtained. In a similar way, the same PCR fragment was cloned into pSFV-1 digested with BamH I, plasmid pSFV-rCT being generated.

To construct plasmid pSFV-S2-9-pac2A-rCT, the cloning vector pSFV-S2-9-pac2A, containing the sequence of vector SFV-S2-9-pac in which the pac gene is fused in phase at its 3' end with the nucleotide sequence encoding the FMDV autoprotease 2A (35), containing at the end of this sequence the cloning site SmaI/XmaI, was generated previously. Briefly, using plasmid pSFV-S2-9-pac as a mold, a crossover PCR was carried out with the external oligonucleotides: SEQ ID NO: 19 and SEQ ID NO: 20, and internal oligonucleotides: SEQ ID NO: 21 and SEQ ID NO: 22, generating a 842 bp DNA fragment which was digested with BamH I and Xma I and was cloned into pSFV-S2-9-pac digested with the same enzymes, thus obtaining plasmid pSFV-S2-9-pac2A. The crossover PCR was carried out for the dual purpose of introducing sequence 2A-XmaI at the 3' end of the pac gene and simultaneously eliminating a XmaI site present inside that gene.

Finally, to generate plasmid pSFV-S2-9-pac2A-rCT, 645 bp PCR fragment containing the rat cardiotrophin gene from plasmid pRSET-rCT (36), using oligonucleotides: SEQ ID NO: 23 and SEQ ID NO: 24 hybridizing with the start and the end of the rCT gene respectively and containing Xma I sites at the ends for making the cloning easier. The PCR fragment was digested with Xma I and was cloned into the Xma I site of pSFV-S2-9-pac2A, obtaining plasmid pSFV-S2-9- pac2A-rCT.

### 9.2 Obtaining stable cell lines producing cardiotrophin and analysis of the cardiotrophin expression

The RNA of each plasmid synthesized *in vitro* (section 9.1) was electroporated in BHK-21 cells. 24 hours after the electroporation, puromycin at 5 µg/ml was added and, when the selected cells reached confluence, the cardiotrophin expression was analyzed in the cell lysates by means of immunoblotting with an antibody specific for cardiotrophin (Figure 15B).

For the experiments of analysis by immunoblotting, lysates of the BHK-21 cells transfected with the SFV vectors of SFV were obtained by means of incubating in a buffer containing 1% Igepal (Sigma, USA), 50 mM Tris HCl, pH 7.6, 150 mM NaCl, 2 mM EDTA and 1 µg/ml PMSF (Sigma, St. Louis, USA), they were clarified by centrifugation for 6 minutes at 6000 rpm in a refrigerated microcentrifuge and were quantified by means of a Bradford analysis. The lysates were analyzed by means of SDS-PAGE in 12% polyacrylamide gels, they were transferred to a nitrocellulose membrane and were incubated with a polyclonal antiserum against murine cardiotrophin CT-1 (R&D Systems) or actin (Sigma, St. Louis, USA) as primary antibodies, respectively. A goat or sheep antiserum specific for rat or rabbit immunoglobulins, respectively, conjugated with HRP as a secondary antibody, was used. The proteins were viewed using Western Lightning Chemiluminiscence Reagent Plus (Perkin Elmer Life Sciences, USA), according to the manufacturer's instructions. To quantify the rCT levels, different amounts of the lysates of the cells expressing rCT were analyzed by immunoblotting and were compared with known amounts of recombinant cardiotrophin, using the Imagequant TL program (Amersham) for that purpose.

The cardiotrophin expression levels in the lines generated with each of the vectors were similar to those obtained in cells electroporated with vector RNA with the wild replicase SFV-rCT (Figure 15B). The rCT expression in the selected lines was approximately 4.3 pg/cell. In the lines generated with vector SFV-S2-9-pac2A-rCT, it was observed that most the rCT had been released from the fusion protein, although there was an undigested fraction which was also detected by immunoblotting.

To analyze the stability of these vectors in the transfected cells, ten successive passages of the cells containing them were carried out in the presence of puromycin for a period of about 20 days and the rCT expression in each passage in cell lysates was determined by means of immunoblotting. In the case of cell lines generated with vector SFV-S2-9-rCT-pac, it was observed that the expression was maintained until passage 5, from which it started to decrease drastically to virtually disappear at passage 11 (Figure 16). This loss of stability, however, did not occur in the lines containing vector SFV-S2-9-pac2A-rCT, in which the expression remained constant for at least 10 passages (Figure 17), which indicates the fusion in phase of the transgene (rCT gene) with the pac gene using the nucleotide sequence encoding the FMDV autoprotease 2A allows considerably increasing the stability of the heterologous protein expression in the lines generated from vector SFV-S2-9.

### EXAMPLE 10

### Obtaining stable cell lines producing human insulin-like growth factor (IGF-I) using vector SFV-S2-9

Plasmids were generated by introducing the human insulin-like growth (IGF-I) gene into pSFV-S2-9-pac in two different environments and they were used to generate cell lines expressing IGF-I. Both the IGF-I expression and the stability of the IGF-I expression were then analyzed for the purpose of identifying stable cell lines expressing IGF-I.

### 10.1 Plasmid construction

For the purpose of generating cell lines expressing IGF-I, plasmids based on SFV-S2-9-pac were generated by introducing IGF-I gene, according to two different embodiments
- plasmid pSFV-S2-9-IGF-pac, in which the pac and IGF-I genes are placed downstream of independent subgenomic promoters (Figure 18); in this plasmid the IGF-I gene was fused with the SFV capsid translation enhancer using the nucleotide sequence encoding FMDV autoprotease 2A as a linker, since this strategy has allowed considerably increasing the heterologous protein expression levels in the wild SFV vector (37); and
- plasmid pSFV-S2-9-pac2A-IGF, constructed according to the general structure described in Figure 8B, containing the pac gene and the IGF-I gene fused downstream of a single subgenomic promoter (Figure 18); in this embodiment the nucleotide sequence encoding FMDV autoprotease 2A (SEQ ID NO:14) (35) was introduced between the two genes to allow the release of the cardiotrophin from the fusion protein (Figure 18).

To construct plasmid pSFV-S2-9-IGF-pac, plasmid pSFVb12A-IGF-IB (38) was digested with BglII+Klenow and SpeI, obtaining 2.3 kb fragment which was ligated with the 11.1 kb fragment obtained from pSFV-S2-9-pac digested with BsmI+T4pol and SpeI. Plasmid pSFVb12A-IGF-IB contains the human IGF-I precursor sequence (IGF-IB) fused at its 5' end with a cassette comprising the sequence encoding the first 34 amino acids of the SFV capsid (minimum translation enhancer or "b1") followed by the nucleotide sequence encoding FMDV autoprotease 2A. Precursor IGF-IB encodes a 195 amino acid preprotein including domains II, B, C, A, D, D, E, Ea Eb and 6 of the IGF-I gene. This preprotein is processed to the mature form of IGF-I which is secreted, losing the aminoterminal II domains and the carboxyterminal domains E, Ea, Eb and 6 (39).

To construct plasmid pSFV-S2-9-pac2A-IGF, plasmid pSFVb12A-IGF-I (38) was digested with XmaI, obtaining a 0.59 kb fragment containing IGF-IB sequence which was ligated with pSFV-S2-9-pac2A (the characteristics of which and the way of obtaining it are described in Example 9.1, in relation to the construction of plasmid pSFV-S2-9-pac2A-rCT) digested previously with XmaI. Clones were then selected in which the 0.59 kb fragment had been introduced in the correct orientation.

### 10.2 Obtaining stable cell lines producing IGF-I and analysis of the IGF-I expression

The RNA of each plasmid synthesized *in vitro* (section 10.1) was electroporated in BHK-21 cells. 24 hours after the electroporation, puromycin at 5 µg/ml was added and, when the selected cells reached confluence, the IGF-I expression was analyzed in the cellular supernatants collected at different times by means of ELISA specific for human IGF-I (Figure 19).

The IGF-I expression was analyzed in cellular supernatants using an ELISA kit specific for measuring free human IGF-I (Free IGF-I, ref DSL-10-9400, Diagnostic Systems laboratories, Webster, Texas, USA). For each of the analyzed passages, 5x10⁵ cells per well were seeded, there was a wait of 4 hours so that the cells were adhered and the medium was changed by adding 1 ml of Glasgow-MEM medium with 5% fetal bovine serum (37) and puromycin at 5 µg/ml. The supernatant was collected after 24, 48 or 72 hours, it was centrifuged at 6,000 rpm for 5 minutes in a refrigerated microfuge to eliminate cellular residues and it was frozen at -80°C until it was analyzed, For the purpose of controlling the amount of cells present in each sample, BHK cells were collected from the same wells in which supernatants had been collected and they were lysated by means of incubating in a buffer containing 1% Igepal (Sigma, USA), 50 mM Tris HCl, pH 7.6, 150 mM NaCl, 2 mM EDTA and 1 µg/ml PMSF (Sigma, St. Louis, USA), they were clarified by centrifugation at 6000 rpm in a refrigerated microcentrifuge and they were quantified by means of a Bradford analysis. The values of the amount of protein obtained by Bradford were very similar in all the analyzed samples.

The results obtained in relation to the IGF-I expression in the collected cellular supernatants showed that, after hours, the IGF-I expression levels in the supernatant of the lines generated with each of the noncytopathic vectors were only between 1.5-2 times lower than those obtained in cells electroporated with RNA of the wild vector SFV-IGF-I (Figure 19). This expression was somewhat greater in the line obtained with vector SFV-S2-9-pac2A-IGF (34.5 pg/cell) with respect to the expression in the line selected with SFV-S2-9-IGF-pac (21.3 pg/cell). The analysis of the IGF-I expression in supernatants taken at longer times demonstrated that IGF-I can be accumulated up to a maximum of 67 and 34 pg/cell in vectors SFV-S2-9-pac2A-IGF and SFV-S2-9-IGF-pac, respectively. These results show that IGF-I is being secreted to the extracellular medium, which is indicating a correct polyprotein processing.

To analyze the stability of these vectors in the transfected cells, 10 successive passages of the cells containing them were carried out in the presence of puromycin for a period of about 20 days and the IGF-I expression in cellular supernatants collected after 24 hours after each of the passages was determined by means of ELISA specific for human IGF-I. In the case of the lines generated with vector SFV-S2-9-IGF-pac, it was observed that the expression was maintained until passage 4, from which it started to decrease drastically, reaching levels that were 80 times less in passage 10 (Figure 20). This loss of stability, did not occur so markedly in the lines containing vector SFV-S2-9-pac2A-IGF, in which the expression remained almost constant, decreasing 4-fold between passages 1 and 10 (Figure 20). This result confirms that the fusion in phase of the transgene with the pac gene using the nucleotide sequence encoding FMDV autoprotease 2A allows considerably increasing the stability of the heterologous protein expression in the lines generated with vector SFV-S2-9.

### LITERATURE

1. Agapov, E. V., I. Frolov, B. D. Lindenbach, B. M. Pragai, S. Schlesinger and C. M. Rice. 1998 Noncytopathic Sindbis virus RNA vectors for heterologous gene expression. Proc. Natl. Acad. Sci. USA. 95: 12989-94.
2. Berglund, P., M. Sjoberg, H. Garoff, G. J. Atkins, B. J. Sheahan and P. Liljeström. 1993. Semliki Forest virus expression system: production of conditionally infectious recombinant particles. Biotechnology (NY) 11: 916-20.
3. Berglund, P., C. Smerdou, M. N. Fleeton, I. Tubulekas and P. Liljeström. 1998. Enhancing immune responses using suicidal DNA vaccines. Nat. Biotechnol. 16: 562-5.
4. Bredenbeek, P. J., I. Frolov, C. M. Rice and S. Schlesinger. 1993. Sindbis virus expression vectors: packaging of RNA replicons by using defective helper RNAs. J. Virol. 67: 6439-46.
5. de la Luna, S. I. Soria, D. Pulido, J. Ortin and A. Jimenez. 1998. Efficient transformation of mammalian cells with constructs containing a puromycin-resistance marker. Gene 62: 121-6.
6.Fazakerley, J. K., A. Boyd, M. L., Mikkola and L. Kaariainen. 2002. A single amino acid change in the nuclear localization sequence of the nsp2 protein affects the neurovirulence of Semliki forest virus. J. Virol. 76: 392-6.
7. Frolov, I., E. Agapov, T. A. Hoffman, Jr., B. M. Pragai, M. Lippa, S. Schlesinger and C. M. Rice. 1999. Selection of RNA replicons capable of persistent noncytopathic replication in mammalian cells. J. Virol. 73: 3854-65.
8. Frolov, 1., E. Frolova and S. Schlesinger. 1997. Sindbis virus replicons and Sindbis virus: assembly of chimeras and of particles deficient in virus RNA. J. Virol. 71: 2819-29.
9. Frolov, I., and S. Schlesinger. 1994. Comparison of the effects of Sindbis virus and Sindbis virus replicons on host cell protein synthesis and cytopathogenicity in BHK cells. J. Virol. 68: 1721-7.
10. Frolov, I., and S. Schlesinger. 1994. Translation of Sindbis virus mRNA: effects of sequences downstream of the initiating codon. J. Virol. 68: 8111-7.
11. Frolova, E. I., R. Z. Fayzulin, S. H. Cook, D. E. Griffin, C. M. Rice and I. Frolov. 2002. Roles of nonstructural protein nsp2 and alfa/beta interferons in determining the outcome of Sindbis virus infection. J. Virol. 76: 11254-64.
12. Garmashova, N., R. Gorchakov, E. Frolova and I. Frolov. 2006. Sindbis virus nonstructural protein nsP2 is cytotoxic and inhibits cellular transcription. J. Virol. 80: 5686-96.
13. Glasgow, G. M., M. M. McGee, B. J. Sheahan and G. J. Atkins. 1997. Death mechanisms in cultured cells infected by Semliki Forest virus. J. Gen. Virol. 78: (Pt: 7): 1559-63.
14. Glasgow, G. M., M. M. McGee, C. J. Tarbatt, D. A. Mooney, B. J. Sheahan and G. J. Atkins. 1998. The Semliki Forest virus vector induces p53-independent apoptosis. J. Gen. Virol. 79 (Pt 10): 2405-10.
15. McInerney, G. M., N. L. Kedersha, R. J. Kaufman, P. Anderson and P. Liljestrom. 2005. Importance of eIF2alpha phosphorylation and stress granule assembly in alphavirus translation regulation. Mol. Biol. Cell. 16:3753-63.
16. Kujala, P. M. Rikkonen, T. Ahola. M. Kelve, M. Saarma and L. Kaariainen. 1997. Monoclonal antibodies specific for Semliki Forest virus replicase protein nsP2. J. Gen. Virol. 78 (Pt: 2): 343-51.
17. Liljeström, P., and H. Garoff. 1994. Expresion of proteins using Semliki Forest virus vectors, pág. 16.20.11-16.20.16. En: F. M. Ausubel, R. Brent, R. E. Kingston, D. D. Moore, J. A. Smith, J. G. Seidman and K. Struhl (ed.), «Current protocols in molecular biology», vol. 2. Greene Publishing Associates and Wiley Interscience, New York, N. Y.
18. Liljeström, P., and H. Garoff. 1991. A new generation of animal cell expression vectors based on the Semliki Forest virus replicon. Biotechnology (NY) 9: 1356-61.
19. Lundström, K. 2003. Alphavirus vectors for vaccine production and gene therapy. Expert Rev. Vaccines 2: 447-59.
20. Lundström, K., A. Abenavoli, A. Malgaroli and M. U. Ehrengruber. 2003. Novel Semliki Forest virus vectors with reduced cytotoxicity and temperature sensitivity for long-term enhancement of transgene expression. Mol. Ther. 7: 202-9.
21. Lundström, K., D. Rotmann, D. Hermann, E. M. Schneider and M. U. Ehrengruber. 2001. Novel mutant Semliki Forest virus vectors: gene expression and localization studies in neuronal cells. Histochem. Cell. Biol. 115: 83-91.
22. Perri, S., D. A. Driver, J. P. Gardner, S. Scherrill, B. A. Belli, T. W. Dubensky, Jr. and J. M. Polo. 2000. Replicon vectors derived from Sindbis virus and Semliki Forest virus that establish persistent replication in host cells. J. Virol. 74: 9802-7.
23. Petrakova, O., E. Volkova, R. Gorchakov, S. Paessler, R. M. Kinney and I. Frolov. 2005. Noncytopathic replication of Venezuelan equine encephalitis virus and eastern equine encephalitis virus replicons in mammalian cells. J. Virol. 79: 7597-608.
24. Polo, J. M., B. A. Belli, D. A. Driver, I. Frolov, S. Sherrill, M. J. Hariharan, K. Townsend, S. Perry, S. J. Mento, D. J. Jolly, S. M. Chang, S. Schlesinger and T. W. Dubensky, Jr. 1999. Stable alphavirus packaging cells lines for Sindbis virus and Semliki Forest virus-derived vectors. Proc. Natl. Acad. Sci. USA. 96: 4598-603.
25. Pushko, P., M. Parker, G. V. Ludwig, N. L. Davis, R. E. Johnston and J. F. Smith. 1997. Replicon-helper systems from attenuated Venezuelan equine encephalitis virus: expression of heterologous genes in vitro an immunization against heterologous pathogens in vivo. Virology 239: 398-401.
26. Rayner, J. O., S. A. Dryga and K. I. Kamrud. 2002. Alphavirus vectors and vaccination. Rev. Med. Virol. 12: 279-96.
27. Rikkonen, M. 1996. Functional significance of the nuclear-targeting and NTP-binding motifs of Semliki Forest virus nonstructural protein nsP2. Virology 218: 352-61.
28. Sjoberg, E. M., M. Suomalainen and H. Garoff. 1994. A significantly improved Semliki Forest virus expression system based on translation enhancer segments from the viral capsid gene. Biotechnology (NY) 12: 1127-31.
29. Smerdou, C., and P. Liljeström. 1999. Two-helper RNA system for production of recombinant Semliki Forest virus particles. J. Virol. 73: 1092-8.
30. Strauss, J. H and E. G. Strauss. 1994. The alphaviruses: gene expression, replication and evolution. Microbiol. Rev. 58: 491-562.
31. Ventoso, I., M. A. Sanz, S. Molina, J. J. Berlanga, L. Carrasco and M. Esteban. 2006. Translational resistance of late alphavirus mRNA to eIF2alpha phosphorylation: a strategy to overcome the antiviral effect of protein kinase PKR. Genes Dev. 20: 87-100.
32. White, C. L., M. Thomson and N. J. Dimmock. 1998. Deletion analysis of a defective interfering Semliki Forest virus RNA genome defines a region in the nsP2 sequence that is required for efficient packaging of the genome into virus particles. J. Virol. 72: 4320-6.
33. Wurm, F., and A. Bernard. 1999. Large-scale transient expression in mammalian cells for recombinant protein production. Curr. Opin. Biotechnol. 10: 156-9.
34. Xiong, C., R. Levis, P. Shen, S. Schlesinger, C. M. Rice and H. V. Huang. 1989. Sindbis virus: an efficient, broad host range vector for gene expression in animal cells. Science 243: 1188-91.
35. Ryan MD and J Drew. Foot-and-mouth disease virus 2A oligopeptide mediated cleavage of an artificial polyprotein.EMBO J. 1994.13:928-33.
36. Iñiguez M, C Berasain, E Martinez-Anso, M Bustos, P Fortes, D Pennica, MA Avila, and J Prieto. 2006 Cardiotrophin-1 defends the liver against ischemia-reperfusion injury and mediates the protective effect of ischemic preconditioning. J Exp Med. 203:2809-15.
37. Rodriguez-Madoz J.R., Prieto J. and Smerdou C. 2005. Semliki Forest virus vectors engineered to express higher IL-12 levels induce efficient elimination of murine colon adenocarcinomas. Molecular Therapy. 12, 153-163.
38. Palencia, B. 2005 (June). "Bioproducción de IGF-I". Ph.D. Thesis. University of Navarre.
39. Lund PK. 1994. Insulin-like growth factor I: molecular biology and relevance to tissue-specific expression and action. Recent Prog Horm Res. 49:125-48.

## Claims

1. A viral vector comprising a replicon of the Semliki Forest virus (SFV), wherein said replicon comprises (i) the nucleotide sequence encoding the SFV replicase enzyme with mutations P718T and R649H in subunit nsp2, (ii) a polynucleotide comprising a selection gene, and (iii) a polynucleotide comprising a nucleotide sequence encoding a heterologous product of interest.

2. A viral vector according to claim 1, comprising SEQ ID NO: 1 and SEQ ID NO: 2.

3. A viral vector according to claim 1, comprising:
a) a polynucleotide comprising the nucleotide sequence encoding a heterologous product of interest, the expression of which is controlled by a first SFV subgenomic promoter (SG1); and
b) a polynucleotide comprising a selection gene, the expression of which is controlled by a second SFV subgenomic promoter (SG2).

4. A viral vector according to claim 3, wherein said first and second SFV subgenomic promoters are identical.

5. A viral vector according to claim 3, wherein said first and second SFV subgenomic promoters are different.

6. A viral vector according to claim 1, comprising, downstream of a subgenomic promoter, a construct comprising the polynucleotide comprising the selection gene and the polynucleotide comprising the nucleotide sequence encoding the heterologous product of interest, fused in phase.

7. A viral vector according to claim 6, wherein said polynucleotide comprising the selection gene is fused in phase with the polynucleotide comprising the nucleotide sequence encoding the heterologous product of interest, by means of a polynucleotide linker.

8. A viral vector according to claim 7, wherein said linker is a polynucleotide comprising the nucleotide sequence encoding a post-translational (auto)proteolytic cleavage site.

9. A viral vector according to claim 8, wherein said linker is a polynucleotide comprising the nucleotide sequence encoding an (auto)protease acting in cis between the proteins resulting from the translation of the selection gene and of the nucleotide sequence encoding the heterologous product of interest.

10. A viral vector according to claim 9, wherein said (auto)protease is the foot and mouth disease virus (FMDV) autoprotease 2A.

11. A viral vector according to claim 6, wherein the 3' end of the polynucleotide comprising the selection gene is fused in phase to the 5' end of the polynucleotide comprising the nucleotide sequence encoding the heterologous product of interest.

12. A viral vector according to claim 6, wherein the 3' end of the polynucleotide comprising the nucleotide sequence encoding the heterologous product of interest is fused in phase to the 5' end of the polynucleotide comprising the selection gene.

13. A stable cell line which can constitutively express heterologous products of interest, **characterized in that** it is a cell line transfected with a viral vector according to any of claims 1 to 12.

14. A method for generating *in vitro* a stable cell line according to claim 13 which can constitutively express heterologous products of interest, which comprises:
I. transfecting cells with a viral vector according to any of claims 1 to 12;
II. selecting the stable cells generated in step I; and
III. growing and maintaining the stable cells.

15. A method for producing *in vitro* a heterologous product of interest which comprises culturing a stable cell line according to claim 21 under conditions allowing the expression of the heterologous product of interest contained in the viral vector used to generate said stable cell line.

## Patentansprüche

1. Viraler Vektor, umfassend ein Replikon des Semliki-Forest-Virus (SFV), wobei das Replikon (i) die Nukleotidsequenz, die für das SFV-Replikase-Enzym mit den Mutationen P718T und R649H in der Untereinheit nsp2 codiert, (ii) ein Polynukleotid, das ein Selektionsgen umfasst, und (iii) ein Polynukleotid, das eine Nukleotidsequenz umfasst, die für ein heterologes Produkt von Interesse codiert, umfasst.

2. Viraler Vektor gemäss Anspruch 1, umfassend SEQ ID NO: 1 und SEQ ID NO: 2.

3. Viraler Vektor gemäss Anspruch 1, umfassend:
(a) ein Polynukleotid, umfassend die Nukleotidsequenz, die für ein heterologes Produkt von Interesse codiert, dessen Expression durch einen ersten subgenomischen Promotor (SG1) von SFV kontrolliert wird;
(b) ein Polynukleotid, umfassend ein Selektionsgen, dessen Expression durch einen zweiten subgenomischen Promotor (SG2) von SFV kontrolliert wird.

4. Viraler Vektor gemäss Anspruch 3, wobei der erste und zweite subgenomische Promotor von SFV identisch sind.

5. Viraler Vektor gemäss Anspruch 3, wobei der erste und zweite subgenomische Promotor von SFV verschieden sind.

6. Viraler Vektor gemäss Anspruch 1, umfassend, stromabwärts eines subgenomischen Promotors, ein Konstrukt, das das Polynukleotid, das das Selektionsgen umfasst, und das Polynukleotid, das die Nukleotidsequenz umfasst, die für das heterologe Produkt von Interesse codiert, in Phase fusioniert umfasst.

7. Viraler Vektor gemäss Anspruch 6, wobei das Polynukleotid, das das Selektionsgen umfasst, mit dem Polynukleotid, das die Nukleotidsequenz umfasst, die für das heterologe Produkt von Interesse codiert, durch einen Polynukleotidlinker in Phase fusioniert ist.

8. Viraler Vektor gemäss Anspruch 7, wobei der Linker ein Polynukleotid ist, das die Nukleotidsequenz umfasst, die für eine post-translationelle (auto)proteolytische Spaltstelle codiert.

9. Viraler Vektor gemäss Anspruch 8, wobei der Linker ein Polynukleotid ist, das die Nukleotidsequenz umfasst, die für eine (Auto)protease codiert, die zwischen den Proteinen, die aus der Translation des Selektionsgens und der Nukleotidsequenz, die für das heterologe Produkt von Interesse codiert, hervorgehen, in cis wirkt.

10. Viraler Vektor gemäss Anspruch 9, wobei die (Auto)protease die Autoprotease 2A des Maul- und Klauenseuche-Virus (FMDV) ist.

11. Viraler Vektor gemäss Anspruch 6, wobei das 3'-Ende des Polynukleotids, das das Selektionsgen umfasst, in Phase an das 5'-Ende des Polynukleotids, das die Nukleotidsequenz umfasst, die für das heterologe Produkt von Interesse codiert, fusioniert ist.

12. Viraler Vektor gemäss Anspruch 6, wobei das 3'-Ende des Polynukleotids, das die Nukleotidsequenz umfasst, die für das heterologe Produkt von Interesse codiert, in Phase an das 5'-Ende des Polynukleotids, das das Selektionsgen umfasst, fusioniert ist.

13. Stabile Zellinie, die heterologe Produkte von Interesse konstitutiv exprimieren kann, **dadurch gekennzeichnet, dass** es eine Zellinie ist, die mit einem viralen Vektor gemäss einem der Ansprüche 1 bis 12 transfiziert ist.

14. Verfahren zur in-vitro-Erzeugung einer stabilen Zellinie gemäss Anspruch 13, die heterologe Produkte von Interesse konstitutiv exprimieren kann, das folgendes umfasst:
I. Transfizieren von Zellen mit einem viralen Vektor gemäss einem der Ansprüche 1 bis 12;
II. Selektieren der in Schritt I. erzeugten stabilen Zellen; und
III. Kultivieren und Beibehalten der stabilen Zellen.

15. Verfahren zur in-vitro-Herstellung eines heterologen Produkts von Interesse, das das Kultivieren einer stabilen Zellinie gemäss Anspruch 21 unter Bedingungen, die die Expression des heterologen Produkts von Interesse, das in dem viralen Vektor enthalten ist, der zur Erzeugung der stabilen Zellinie verwendet wird, ermöglichen, umfasst.

## Revendications

1. Vecteur viral comprenant un réplicon du virus de la forêt de Semliki (VFS), dans lequel ledit réplicon comprend (i) la séquence nucléotidique codant pour l'enzyme réplicase du VFS avec des mutations P718T et R649H dans la sous-unité nsp2, (ii) un polynucléotide comprenant un gène de sélection, et (iii) un polynucléotide comprenant une séquence nucléotidique codant pour un produit hétérologue d'intérêt.

2. Vecteur viral selon la revendication 1, comprenant SEQ ID NO: 1 et SEQ ID NO: 2.

3. Vecteur viral selon la revendication 1, comprenant :
a) un polynucléotide comprenant la séquence nucléotidique codant pour un produit hétérologue d'intérêt, dont l'expression est contrôlée par un premier promoteur subgénomique (SG1) du VFS ; et
b) un polynucléotide comprenant un gène de sélection, dont l'expression est contrôlée par un second promoteur subgénomique (SG2) du VFS.

4. Vecteur viral selon la revendication 3, dans lequel lesdits premier et second promoteurs subgénomiques du VFS sont identiques.

5. Vecteur viral selon la revendication 3, dans lequel lesdits premier et second promoteurs subgénomiques du VFS sont différents.

6. Vecteur viral selon la revendication 1, comprenant, en aval d'un promoteur subgénomique, une construction comprenant le polynucléotide comprenant le gène de sélection et le polynucléotide comprenant la séquence nucléotidique codant pour le produit hétérologue d'intérêt, fusionnés en phase.

7. Vecteur viral selon la revendication 6, dans lequel ledit polynucléotide comprenant le gène de sélection est fusionné en phase avec le polynucléotide comprenant la séquence nucléotidique codant pour le produit hétérologue d'intérêt, au moyen d'un lieur polynucléotidique.

8. Vecteur viral selon la revendication 7, dans lequel ledit lieur est un polynucléotide comprenant la séquence nucléotidique codant pour un site de clivage (auto)protéolytique post-traductionnel.

9. Vecteur viral selon la revendication 8, dans lequel ledit lieur est un polynucléotide comprenant la séquence nucléotidique codant pour une (auto)protéase agissant en cis entre les protéines résultant de la traduction du gène de sélection et de la séquence nucléotidique codant pour le produit hétérologue d'intérêt.

10. Vecteur viral selon la revendication 9, dans lequel ladite (auto)protéase est l'autoprotéase 2A du virus de la fièvre aphteuse.

11. Vecteur viral selon la revendication 6, dans lequel l'extrémité 3' du polynucléotide comprenant le gène de sélection est fusionnée en phase avec l'extrémité 5' du polynucléotide comprenant la séquence nucléotidique codant pour le produit hétérologue d'intérêt.

12. Vecteur viral selon la revendication 6, dans lequel l'extrémité 3' du polynucléotide comprenant la séquence nucléotidique codant pour le produit hétérologue d'intérêt est fusionnée en phase avec l'extrémité 5' du polynucléotide comprenant le gène de sélection.

13. Lignée de cellules stables qui peut exprimer de manière constitutive des produits hétérologues d'intérêt, **caractérisée en ce qu'**elle est une lignée de cellules transfectée avec un vecteur viral selon l'une quelconque des revendications 1 à 12.

14. Méthode pour générer *in vitro* une lignée de cellules stables selon la revendication 13 qui peut exprimer de manière constitutive des produits hétérologues d'intérêt, qui comprend :
I. la transfection de cellules avec un vecteur viral selon l'une quelconque des revendications 1 à 12 ;
II. la sélection des cellules stables générées à l'étape I ; et
III. la croissance et le maintien des cellules stables.

15. Méthode pour produire *in vitro* un produit hétérologue d'intérêt qui comprend la culture d'une lignée de cellules stables selon la revendication 21 dans des conditions permettant l'expression du produit hétérologue d'intérêt contenu dans le vecteur viral utilisé pour générer ladite lignée de cellules stables.


### SEQUENCE LISTING

<110> PROYECTO DE BIOMEDICINA CIMA, S.L.
<120> VIRAL VECTOR AND ITS APPLICATIONS
<130> P3499EPPC
<140> EP 07858264.0
   <141> 2007-11-28
<150> ES P200603036
   <151> 2006-11-28
<150> ES P200700882
   <151> 2007-04-03
<160> 24
<170> PatentIn version 3.3
<210> 1
   <211> 7382
   <212> DNA
   <213> Semliki Forest virus
<220>
   <221> 5'UTR
   <222> (1)..(86)
   <223> Non-translated 5' UTR end including the sequences necessary for replication
<220>
   <221> misc feature
   <222> (87)..(89)
   <223> Replicase translation initiation triplet
<220>
   <221> misc feature
   <222> (3642)..(3644)
   <223> Triplet corresponding to mutation R649H
<220>
   <221> misc feature
   <222> (3849)..(3851)
   <223> Triplet corresponding to mutation P718T
<220>
   <221> misc feature
   <222> (7351)..(7374)
   <223> Overlapped viral subgenomic promoter
<220>
   <221> misc_feature
   <222> (7380)..(7382)
   <223> Replicase translation termination triplet
<400> 1
   gatggcggat gtgtgacata cacgacgcca aaagattttg ttccagctcc tgccacctcc    60
   gctacgcgag agattaacca cccacgatgg ccgccaaagt gcatgttgat attgaggctg   120
   acagcccatt catcaagtct ttgcagaagg catttccgtc gttcgaggtg gagtcattgc   180
   aggtcacacc aaatgaccat gcaaatgcca gagcattttc gcacctggct accaaattga   240
   tcgagcagga gactgacaaa gacacactca tcttggatat cggcagtgcg ccttccagga   300
   gaatgatgtc tacgcacaaa taccactgcg tatgccctat gcgcagcgca gaagaccccg   360
   aaaggctcga tagctacgca aagaaactgg cagcggcctc cgggaaggtg ctggatagag   420
   agatcgcagg aaaaatcacc gacctgcaga ccgtcatggc tacgccagac gctgaatctc   480
   ctaccttttg cctgcataca gacgtcacgt gtcgtacggc agccgaagtg gccgtatacc   540
   aggacgtgta tgctgtacat gcaccaacat cgctgtacca tcaggcgatg aaaggtgtca   600
   gaacggcgta ttggattggg tttgacacca ccccgtttat gtttgacgcg ctagcaggcg   660
   cgtatccaac ctacgccaca aactgggccg acgagcaggt gttacaggcc aggaacatag   720
   gactgtgtgc agcatccttg actgagggaa gactcggcaa actgtccatt ctccgcaaga   780
   agcaattgaa accttgcgac acagtcatgt tctcggtagg atctacattg tacactgaga   840
   gcagaaagct actgaggagc tggcacttac cctccgtatt ccacctgaaa ggtaaacaat   900
   cctttacctg taggtgcgat accatcgtat catgtgaagg gtacgtagtt aagaaaatca   960
   ctatgtgccc cggcctgtac ggtaaaacgg tagggtacgc cgtgacgtat cacgcggagg  1020
   gattcctagt gtgcaagacc acagacactg tcaaaggaga aagagtctca ttccctgtat  1080
   gcacctacgt cccctcaacc atctgtgatc aaatgactgg catactagcg accgacgtca  1140
   caccggagga cgcacagaag ttgttagtgg gattgaatca gaggatagtt gtgaacggaa  1200
   gaacacagcg aaacactaac acgatgaaga actatctgct tccgattgtg gccgtcgcat  1260
   ttagcaagtg ggcgagggaa tacaaggcag accttgatga tgaaaaacct ctgggtgtcc  1320
   gagagaggtc acttacttgc tgctgcttgt gggcatttaa aacgaggaag atgcacacca  1380
   tgtacaagaa accagacacc cagacaatag tgaaggtgcc ttcagagttt aactcgttcg  1440
   tcatcccgag cctatggtct acaggcctcg caatcccagt cagatcacgc attaagatgc  1500
   ttttggccaa gaagaccaag cgagagttaa tacctgttct cgacgcgtcg tcagccaggg  1560
   atgctgaaca agaggagaag gagaggttgg aggccgagct gactagagaa gccttaccac  1620
   ccctcgtccc catcgcgccg gcggagacgg gagtcgtcga cgtcgacgtt gaagaactag  1680
   agtatcacgc aggtgcaggg gtcgtggaaa cacctcgcag cgcgttgaaa gtcaccgcac 1740
   agccgaacga cgtactacta ggaaattacg tagttctgtc cccgcagacc gtgctcaaga 1800
   gctccaagtt ggcccccgtg caccctctag cagagcaggt gaaaataata acacataacg 1860
   ggagggccgg cggttaccag gtcgacggat atgacggcag ggtcctacta ccatgtggat 1920
   cggccattcc ggtccctgag tttcaagctt tgagcgagag cgccactatg gtgtacaacg 1980
   aaagggagtt cgtcaacagg aaactatacc atattgccgt tcacggaccg tcgctgaaca 2040
   ccgacgagga gaactacgag aaagtcagag ctgaaagaac tgacgccgag tacgtgttcg 2100
   acgtagataa aaaatgctgc gtcaagagag aggaagcgtc gggtttggtg ttggtgggag 2160
   agctaaccaa ccccccgttc catgaattcg cctacgaagg gctgaagatc aggccgtcgg 2220
   caccatataa gactacagta gtaggagtct ttggggttcc gggatcaggc aagtctgcta 2280
   ttattaagag cctcgtgacc aaacacgatc tggtcaccag cggcaagaag gagaactgcc 2340
   aggaaatagt taacgacgtg aagaagcacc gcgggaaggg gacaagtagg gaaaacagtg 2400
   actccatcct gctaaacggg tgtcgtcgtg ccgtggacat cctatatgtg gacgaggctt 2460
   tcgcttgcca ttccggtact ctgctggccc taattgctct tgttaaacct cggagcaaag 2520
   tggtgttatg cggagacccc aagcaatgcg gattcttcaa tatgatgcag cttaaggtga 2580
   acttcaacca caacatctgc actgaagtat gtcataaaag tatatccaga cgttgcacgc 2640
   gtccagtcac ggccatcgtg tctacgttgc actacggagg caagatgcgc acgaccaacc 2700
   cgtgcaacaa acccataatc atagacacca caggacagac caagcccaag ccaggagaca 2760
   tcgtgttaac atgcttccga ggctgggcaa agcagctgca gttggactac cgtggacacg 2820
   aagtcatgac agcagcagca tctcagggcc tcacccgcaa aggggtatac gccgtaaggc 2880
   agaaggtgaa tgaaaatccc ttgtatgccc ctgcgtcgga gcacgtgaat gtactgctga 2940
   cgcgcactga ggataggctg gtgtggaaaa cgctggccgg cgatccctgg attaaggtcc 3000
   tatcaaacat tccacagggt aactttacgg ccacattgga agaatggcaa gaagaacacg 3060
   acaaaataat gaaggtgatt gaaggaccgg ctgcgcctgt ggacgcgttc cagaacaaag 3120
   cgaacgtgtg ttgggcgaaa agcctggtgc ctgtcctgga cactgccgga atcagattga 3180
   cagcagagga gtggagcacc ataattacag catttaagga ggacagagct tactctccag 3240
   tggtggcctt gaatgaaatt tgcaccaagt actatggagt tgacctggac agtggcctgt 3300
   tttctgcccc gaaggtgtcc ctgtattacg agaacaacca ctgggataac agacctggtg   3360
   gaaggatgta tggattcaat gccgcaacag ctgccaggct ggaagctaga cataccttcc   3420
   tgaaggggca gtggcatacg ggcaagcagg cagttatcgc agaaagaaaa atccaaccgc 3480
   tttctgtgct ggacaatgta attcctatca accgcaggct gccgcacgcc ctggtggctg 3540
   agtacaagac ggttaaaggc agtagggttg agtggctggt caataaagta agagggtacc 3600
   acgtcctgct ggtgagtgag tacaacctgg ctttgcctcg acacagggtc acttggttgt 3660
   caccgctgaa tgtcacaggc gccgataggt gctacgacct aagtttagga ctgccggctg 3720
   acgccggcag gttcgacttg gtctttgtga acattcacac ggaattcaga atccaccact 3780
   accagcagtg tgtcgaccac gccatgaagc tgcagatgct tgggggagat gcgctacgac 3840
   tgctaaaaac gggcggcatc ttgatgagag cttacggata cgccgataaa atcagcgaag 3900
   ccgttgtttc ctccttaagc agaaagttct cgtctgcaag agtgttgcgc ccggattgtg 3960
   tcaccagcaa tacagaagtg ttcttgctgt tctccaactt tgacaacgga aagagaccct 4020
   ctacgctaca ccagatgaat accaagctga gtgccgtgta tgccggagaa gccatgcaca 4080
   cggccgggtg tgcaccatcc tacagagtta agagagcaga catagccacg tgcacagaag 4140
   cggctgtggt taacgcagct aacgcccgtg gaactgtagg ggatggcgta tgcagggccg 4200
   tggcgaagaa atggccgtca gcctttaagg gagcagcaac accagtgggc acaattaaaa 4260
   cagtcatgtg cggctcgtac cccgtcatcc acgctgtagc gcctaatttc tctgccacga 4320
   ctgaagcgga aggggaccgc gaattggccg ctgtctaccg ggcagtggcc gccgaagtaa 4380
   acagactgtc actgagcagc gtagccatcc cgctgctgtc cacaggagtg ttcagcggcg 4440
   gaagagatag gctgcagcaa tccctcaacc atctattcac agcaatggac gccacggacg 4500
   ctgacgtgac catctactgc agagacaaaa gttgggagaa gaaaatccag gaagccattg 4560
   acatgaggac ggctgtggag ttgctcaatg atgacgtgga gctgaccaca gacttggtga 4620
   gagtgcaccc ggacagcagc ctggtgggtc gtaagggcta cagtaccact gacgggtcgc 4680
   tgtactcgta ctttgaaggt acgaaattca accaggctgc tattgatatg gcagagatac 4740
   tgacgttgtg gcccagactg caagaggcaa acgaacagat atgcctatac gcgctgggcg 4800
   aaacaatgga caacatcaga tccaaatgtc cggtgaacga ttccgattca tcaacacctc 4860
   ccaggacagt gccctgcctg tgccgctacg caatgacagc agaacggatc gcccgcctta 4920
   ggtcacacca agttaaaagc atggtggttt gctcatcttt tcccctcccg aaataccatg 4980
   tagatggggt gcagaaggta aagtgcgaga aggttctcct gttcgacccg acggtacctt 5040
   cagtggttag tccgcggaag tatgccgcat ctacgacgga ccactcagat cggtcgttac 5100
   gagggtttga cttggactgg accaccgact cgtcttccac tgccagcgat accatgtcgc 5160
   tacccagttt gcagtcgtgt gacatcgact cgatctacga gccaatggct cccatagtag 5220
   tgacggctga cgtacaccct gaacccgcag gcatcgcgga cctggcggca gatgtgcacc 5280
   ctgaacccgc agaccatgtg gacctcgaga acccgattcc tccaccgcgc ccgaagagag 5340
   ctgcatacct tgcctcccgc gcggcggagc gaccggtgcc ggcgccgaga aagccgacgc 5400
   ctgccccaag gactgcgttt aggaacaagc tgcctttgac gttcggcgac tttgacgagc 5460
   acgaggtcga tgcgttggcc tccgggatta ctttcggaga cttcgacgac gtcctgcgac 5520
   taggccgcgc gggtgcatat attttctcct cggacactgg cagcggacat ttacaacaaa 5580
   aatccgttag gcagcacaat ctccagtgcg cacaactgga tgcggtccag gaggagaaaa 5640
   tgtacccgcc aaaattggat actgagaggg agaagctgtt gctgctgaaa atgcagatgc 5700
   acccatcgga ggctaataag agtcgatacc agtctcgcaa agtggagaac atgaaagcca 5760
   cggtggtgga caggctcaca tcgggggcca gattgtacac gggagcggac gtaggccgca 5820
   taccaacata cgcggttcgg tacccccgcc ccgtgtactc ccctaccgtg atcgaaagat 5880
   tctcaagccc cgatgtagca atcgcagcgt gcaacgaata cctatccaga aattacccaa 5940
   cagtggcgtc gtaccagata acagatgaat acgacgcata cttggacatg gttgacgggt 6000
   cggatagttg cttggacaga gcgacattct gcccggcgaa gctccggtgc tacccgaaac 6060
   atcatgcgta ccaccagccg actgtacgca gtgccgtccc gtcacccttt cagaacacac 6120
   tacagaacgt gctagcggcc gccaccaaga gaaactgcaa cgtcacgcaa atgcgagaac 6180
   tacccaccat ggactcggca gtgttcaacg tggagtgctt caagcgctat gcctgctccg 6240
   gagaatattg ggaagaatat gctaaacaac ctatccggat aaccactgag aacatcacta 6300
   cctatgtgac caaattgaaa ggcccgaaag ctgctgcctt gttcgctaag acccacaact 6360
   tggttccgct gcaggaggtt cccatggaca gattcacggt cgacatgaaa cgagatgtca 6420
   aagtcactcc agggacgaaa cacacagagg aaagacccaa agtccaggta attcaagcag 6480
   cggagccatt ggcgaccgct tacctgtgcg gcatccacag ggaattagta aggagactaa 6540
   atgctgtgtt acgccctaac gtgcacacat tgtttgatat gtcggccgaa gactttgacg 6600
   cgatcatcgc ctctcacttc cacccaggag acccggttct agagacggac attgcatcat 6660
   tcgacaaaag ccaggacgac tccttggctc ttacaggttt aatgatcctc gaagatctag 6720
   gggtggatca gtacctgctg gacttgatcg aggcagcctt tggggaaata tccagctgtc 6780
   acctaccaac tggcacgcgc ttcaagttcg gagctatgat gaaatcgggc atgtttctga   6840
   ctttgtttat taacactgtt ttgaacatca ccatagcaag cagggtactg gagcagagac   6900
   tcactgactc cgcctgtgcg gccttcatcg gcgacgacaa catcgttcac ggagtgatct   6960
   ccgacaagct gatggcggag aggtgcgcgt cgtgggtcaa catggaggtg aagatcattg   7020
   acgctgtcat gggcgaaaaa cccccatatt tttgtggggg attcatagtt tttgacagcg   7080
   tcacacagac cgcctgccgt gtttcagacc cacttaagcg cctgttcaag ttgggtaagc   7140
   cgctaacagc tgaagacaag caggacgaag acaggcgacg agcactgagt gacgaggtta   7200
   gcaagtggtt ccggacaggc ttgggggccg aactggaggt ggcactaaca tctaggtatg   7260
   aggtagaggg ctgcaaaagt atcctcatag ccatggccac cttggcgagg gacattaagg   7320
   cgtttaagaa attgagagga cctgttatac acctctacgg cggtcctaga ttggtgcgtt   7380
   aa                                                                  7382
<210> 2
   <211> 841
   <212> DNA
   <213> Semliki Forest virus
<220>
   <221> 3'UTR
   <222> (1)..(841)
   <223> Non-translated SFV 3' end including the sequences necessary for replication
<220>
   <221> misc feature
   <222> (772)..(841)
   <223> Tail of polyadenines (Poly A)
<400> 2
   attacatccc tacgcaaacg ttttacggcc gccggtggcg cccgcgcccg gcggcccgtc     60
   cttggccgtt gcaggccact ccggtggctc ccgtcgtccc cgacttccag gcccagcaga    120
   tgcagcaact catcagcgcc gtaaatgcgc tgacaatgag acagaacgca attgctcctg    180
   ctaggcctcc caaaccaaag aagaagaaga caaccaaacc aaagccgaaa acgcagccca    240
   agaagatcaa cggaaaaacg cagcagcaaa agaagaaaga caagcaagcc gacaagaaga    300
   agaagaaacc cggaaaaaga gaaagaatgt gcatgaagat tgaaaatgac tgtatcttcg    360
   tatgcggcta gccacagtaa cgtagtgttt ccagacatgt cgggcaccgc actatcatgg    420
   gtgcagaaaa tctcgggtgg tctgggggcc ttcgcaatcg gcgctatcct ggtgctggtt    480
   gtggtcactt gcattgggct ccgcagataa gttagggtag gcaatggcat tgatatagca    540
   agaaaattga aaacagaaaa agttagggta agcaatggca tataaccata actgtataac    600
   ttgtaacaaa gcgcaacaag acctgcgcaa ttggccccgt ggtccgcctc acggaaactc    660
   ggggcaactc atattgacac attaattggc aataattgga agcttacata agcttaattc    720
   gacgaataat tggattttta ttttattttg caattggttt ttaatatttc caaaaaaaaa    780
   aaaaaaaaaa aaaaaaaaaa aaaaaaaaaa aaaaaaaaaa aaaaaaaaaa aaaaaaaaaa    840
   a                                                                    841
<210> 3
   <211> 21
   <212> DNA
   <213> Artificial
<220>
   <223> Primer SF3669-VS
<400> 3
   aatgtcacag gcgccgatag g 21
<210> 4
   <211> 21
   <212> DNA
   <213> Artificial
<220>
   <223> Primer SF4096-RS
<400> 4
   ggtgcacacc cggccgtgtg c 21
<210> 5
   <211> 37
   <212> DNA
   <213> Artificial
<220>
   <223> Primer mutS2-VS
<400> 5
   gactgctaaa aacgggcggc agcctcttga tgagagc 37
<210> 6
   <211> 38
   <212> DNA
   <213> Artificial
<220>
   <223> Primer mutS2-RS
<400> 6
   aagaggctgc cgcccgtttt tagcagtcgt agcgcatc 38
<210> 7
   <211> 37
   <212> DNA
   <213> Artificial
<220>
   <223> Primer mutS3-VS
<400> 7
   gactgctaaa atttggcggc agcctcttga tgagagc 37
<210> 8
   <211> 38
   <212> DNA
   <213> Artificial
<220>
   <223> Primer mutS3-RS
<400> 8
   aagaggctgc cgccaaattt tagcagtcgt agcgcatc 38
<210> 9
   <211> 18
   <212> DNA
   <213> Artificial
<220>
   <223> Primer SF1947-VS
<400> 9
   cggtccctga gtttcaag 18
<210> 10
   <211> 71
   <212> DNA
   <213> Artificial
<220>
   <223> Primer SF3623-S29-RS
<400> 10
   agcacctatc ggcgcctgtg acattcagcg gtgacaacca agtgaccctg tgtcgaggca      60
   aagccaggtt g                                                           71
<210> 11
   <211> 34
   <212> DNA
   <213> Artificial
<220>
   <223> Primer
<400> 11
   tctcgcactt taccttctgc accccatcta catg 34
<210> 12
   <211> 35
   <212> DNA
   <213> Artificial
<220>
   <223> Primer
<400> 12
   cacagacact gtcaaaggag aaagagtctc attcc 35
<210> 13
   <211> 21
   <212> DNA
   <213> Artificial
<220>
   <223> Primer
<400> 13
   gaattctgtg tattaacgca c 21
<210> 14
   <211> 51
   <212> DNA
   <213> Aphthovirus
<400> 14
   aattttgacc ttcttaagct tgcgggagac gtcgagtcca accctgggcc c 51
<210> 15
   <211> 92
   <212> DNA
   <213> Artificial
<220>
   <223> Primer
<400> 15
   gatcctaggg ccctcgcgat tcgaataatt gattaattat acacctctac ggcggtccta 60
   gattggtgcg ttaatacaca gaattctgat tc 92
<210> 16
   <211> 92
   <212> DNA
   <213> Artificial
<220>
   <223> Primer
<400> 16
   gatcgaatca gaattctgtg tattaacgca ccaatctagg accgccgtag aggtgtataa 60
   ttaatcaatt attcgaatcg cgagggccct ag 92
<210> 17
   <211> 36
   <212> DNA
   <213> Artificial
<220>
   <223> Primer
<400> 17
   gggggatcct agcaccatga gccagaggga gggaag 36
<210> 18
   <211> 34
   <212> DNA
   <213> Artificial
<220>
   <223> Primer
<400> 18
   gggggatcca catatgtcag gcaacgcccc ctgg 34
<210> 19
   <211> 20
   <212> DNA
   <213> Artificial
<220>
   <223> Primer
<400> 19
   ttggcgaggg acattaaggc 20
<210> 20
   <211> 91
   <212> DNA
   <213> Artificial
<220>
   <223> Primer
<400> 20
   cactggatat ctcacccggg cccagggttg gactcgacgt ctcccgcaag cttaagaagg 60
   tcaaaattgg caccgggctt gcgggtcatg c 91
<210> 21
   <211> 41
   <212> DNA
   <213> Artificial
<220>
   <223> Primer
<400> 21
   ggcgagggtg cgtacggccc gcgggacgtc gtcgcgggtg g 41
<210> 22
   <211> 40
   <212> DNA
   <213> Artificial
<220>
   <223> Primer
<400> 22
   gccacccgcg acgacgtccc gcgggccgta cgcaccctcg 40
<210> 23
   <211> 36
   <212> DNA
   <213> Artificial
<220>
   <223> Primer
<400> 23
   cgtatacgta cccgggatga gccagaggga gggaag 36
<210> 24
   <211> 34
   <212> DNA
   <213> Artificial
<220>
   <223> Primer
<400> 24
   cgtatacgta cccgggtcag gcaacgcccc ctgg 34